# EUROPEAN PATENT SPECIFICATION

(11) **EP 3 790 619 B1**
(45) Date of publication and mention of the grant of the patent: **09.12.2026**
(21) Application number: 19800094.5
(22) Date of filing: 08.05.2019
(51) Int. Cl.: A61M 39/10, A61B 17/34, A61M 29/02

(54) **COUPLING MECHANISMS FOR MEDICAL DEVICES**
KUPPLUNGSMECHANISMEN FÜR MEDIZINPRODUKTE
SYSTÈME D'ACCOUPLEMENT POUR DISPOSITIFS MÉDICAUX

(30) Priority: 08.05.2018 US 201862668700 P
(43) Date of publication of application: 17.03.2021
(60) Divisional application: 26215798.5
(73) Proprietor: Boston Scientific Medical Device Limited, Galway (IE)
(72) Inventor: ARNETT, Jeffery, Gilbert, Arizona 85297 (US); CHEHADE, Moussa, Toronto, Ontario M1R 4W7 (CA); URBANSKI, John Paul, Toronto, Ontario M4L 2N7 (CA)
(74) Representative: Pfenning, Meinig & Partner mbB
(86) International application number: PCT/IB2019/053755
(87) International publication number: WO 2019/215623

(56) References cited:
- WO-A1-2006/130378
- WO-A1-2016/018998
- CN-U- 204 601 342
- CN-U- 204 601 342
- JP-A- 2010 137 043
- US-A- 3 990 727
- US-A- 5 090 747
- US-A- 5 573 279
- US-A1- 2006 264 911
- US-A1- 2006 267 341

## Description

The disclosure relates to systems and methods that incorporate coupling mechanisms that allow for coupling two mating member such as two medical devices such as introducers, sheaths, dilators and the like for a part of the procedure. More specifically, the disclosure relates to releasable coupling mechanisms, specifically snap-fit mechanisms, to allow for releasably coupling two medical devices such as a dilator and sheaths for a part of the procedure.

Document CN 204 601 342 U describes device for locking and unlocking a catheter sheath and a dilator. The catheter sheath comprises a sheath seat and a sheath tube connected to the sheath seat. The dilator comprises a dilator seat and a dilator tube connected to the dilator seat, the dilator tube passing through the sheath seat and inserted into the sheath tube. The device is used to lock and unlock the sheath seat and the dilator seat, and comprises a snap-on mechanism and a release mechanism arranged between the sheath seat and the dilator seat. The snap-on mechanism is suitable for snapping the dilator seat with the sheath seat during the process of pushing the dilator tube into the sheath tube. The release mechanism is suitable for causing the dilator to generate a moving force relative to the sheath seat during the process of rotating the dilator seat relative to the sheath seat, thereby forcing the sheath seat and the dilator seat to be released.

Document US 20067264911 A1 discloses a connector for coupling a medical tubing (e.g., a catheter) to an implantable medical device. The connector may include a retaining member surrounding an optional housing. The retaining member may have at least one locking finger that extends through at least one corresponding window in the housing and selectively engages a stem of the medical device to retain the end of the catheter relative thereto. Application of a compressive force applied to the retaining member may cause the locking finger(s) to withdraw from the stem and release the catheter.

Document WO 2006/130378 A1 describes an access port for a medical fluid container. The access port includes: (i) a shell; (ii) a perforator located within the shell, the perforator including an end configured to pierce a medical fluid container; and (iii) a safety tab connected to the perforator, the safety tab initially preventing the perforator from piercing the medical fluid container, the safety tab manually removable to enable the perforator to pierce the medical fluid container. The shell can include a pair of hingedly moving arms and members connected hingedly to the arms, the members operable to push the perforator towards the medical fluid container when the arms are pushed. The safety tab can include a plurality of frangible fixtures, the fixtures collectively providing a suitably high tamper resistance force, the fixtures individually providing a suitably low tab removal force.

### SUMMARY

The invention is defined in claim 1. Further aspects and preferred embodiments are defined in the dependent claims. Aspects, embodiments and examples of the present disclosure which do not fall under the scope of the appended claims do not form part of the claimed invention and are provided for illustrative purposes.

### BRIEF DESCRIPTION OF THE DRAWINGS

In order that the invention may be readily understood, embodiments of the invention are illustrated by way of examples in the accompanying drawings, in which:
Fig. 1A is a perspective view of a hub for a medical device, comprising a coupling mechanism in accordance with an embodiment of the present invention and further showing a second device at least partially inserted into the hub;
Fig. 1B shows a cross-sectional view of the hub of Fig. IA and the coupling mechanism incorporated therein, in accordance with an embodiment of the present invention;
Fig. 1C is a perspective view of the hub, showing a coupling mechanism comprising guides and co-operating features for enabling coupling of two medical devices in accordance with an embodiment of the present invention;
Fig. 1D is a side view of a device, for example a dilator, inserted into the hub of a second medical device, for example a sheath, wherein the sheath comprises a coupling mechanism in accordance with an embodiment of the present invention;
Fig. 2A(i) is a perspective view of a hub with a coupling mechanism, showing another device about to be inserted into the hub;

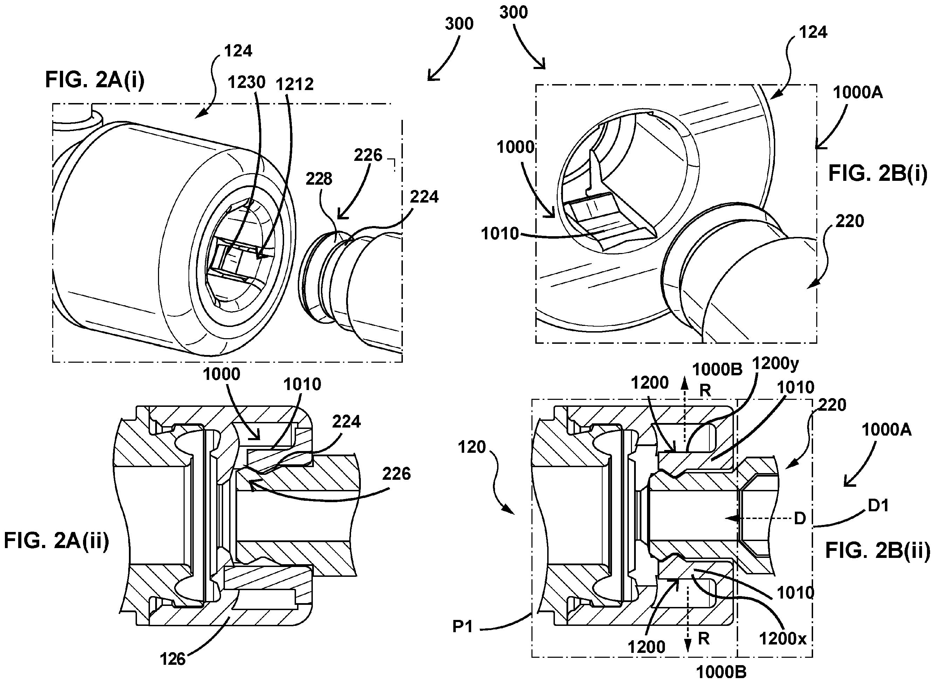

Fig. 2A(ii) is a cross sectional view of the hub ofFigure 2A(i) , showing the other device partially inserted through the coupling mechanism;
Fig. 2B(i) is a perspective view of a hub with a coupling mechanism, showing another device about to be inserted into the hub;
Fig. 2B(ii) is a cross sectional view of the hub of Figure 2B(i), showing the other device partially inserted through the coupling mechanism;

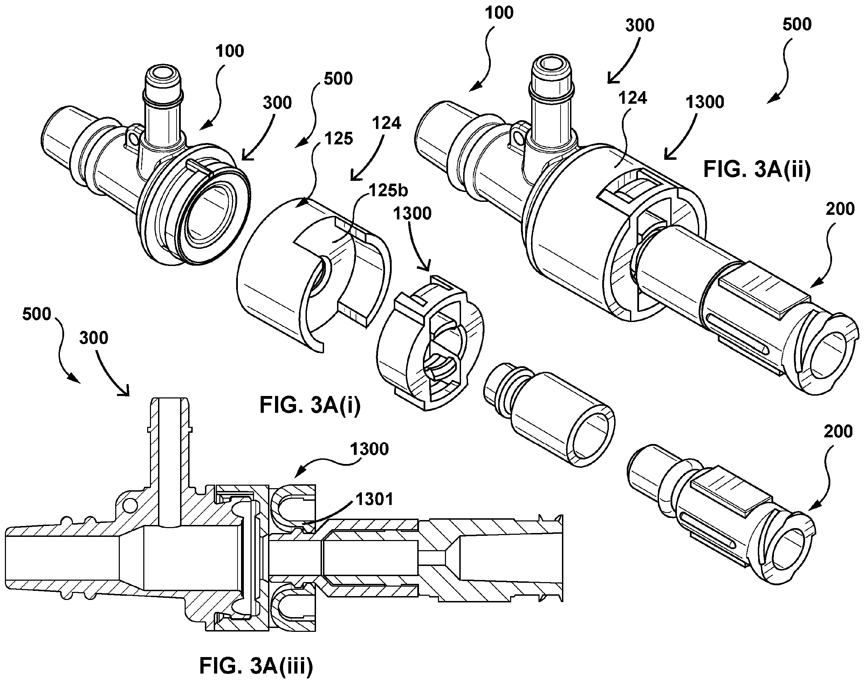

Figures 3A(i), 3A(ii) and 3A(iii) are, respectively, an exploded view, a perspective view and a cross section through a hub containing an embodiment of a coupling mechanism of the present invention;

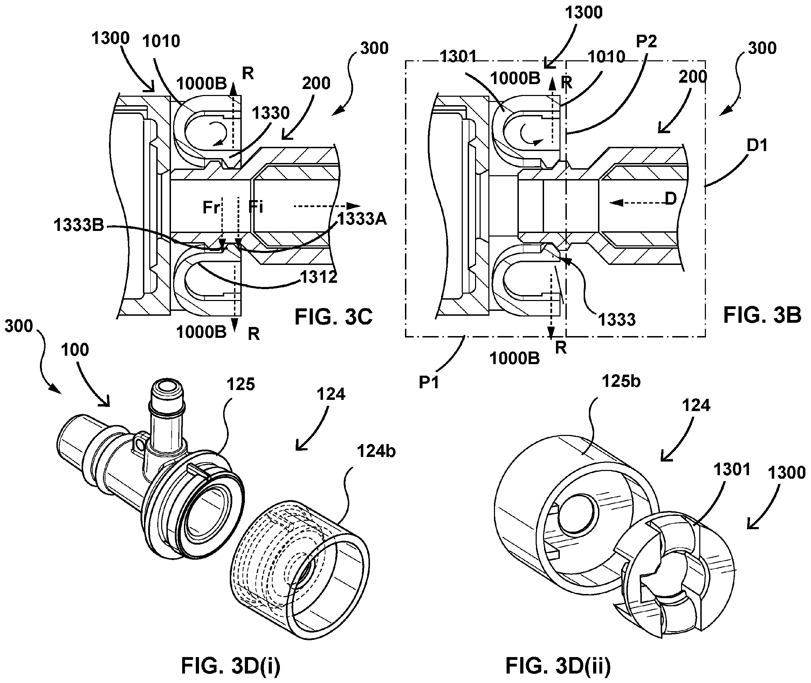

Figures 3B and 3C are, respectively, cross sectional views of a device being inserted through an embodiment of a coupling mechanism and the device being retracted therefrom;
Figures 3D(i) and 3D(ii) are perspective exploded views illustrating various features of a hub and coupling mechanism of the present invention;

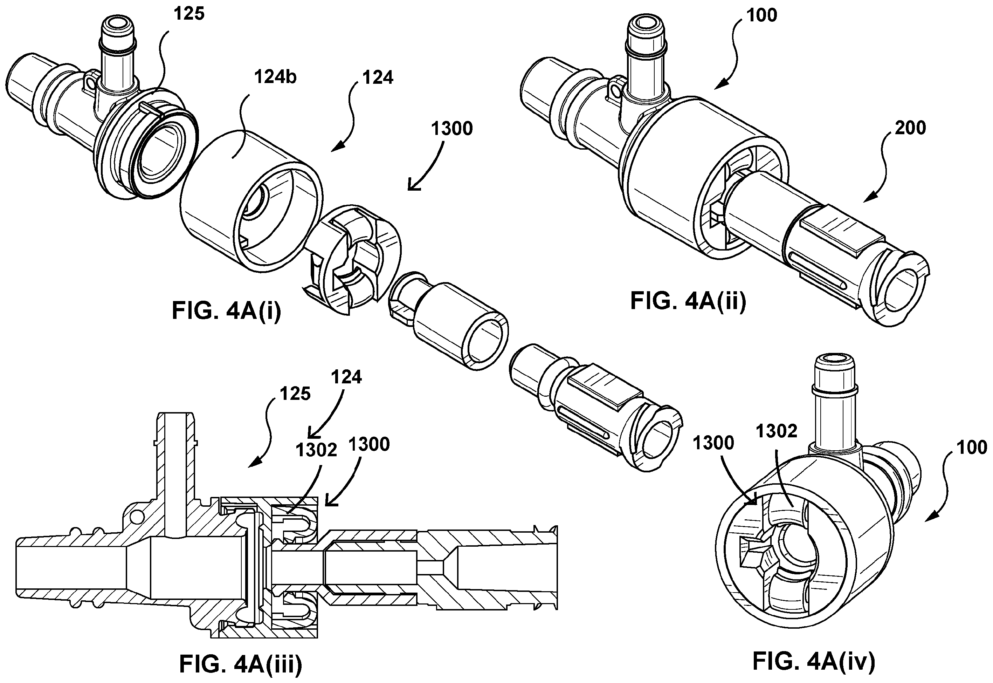

Figures 4A(i), 4A(ii) and 4A(iii) are, respectively, an exploded view, a perspective view and a cross section through a hub containing an embodiment of a coupling mechanism of the present invention;
Figure 4A(iv) is a perspective view of an embodiment of a hub and coupling mechanism of the present invention;

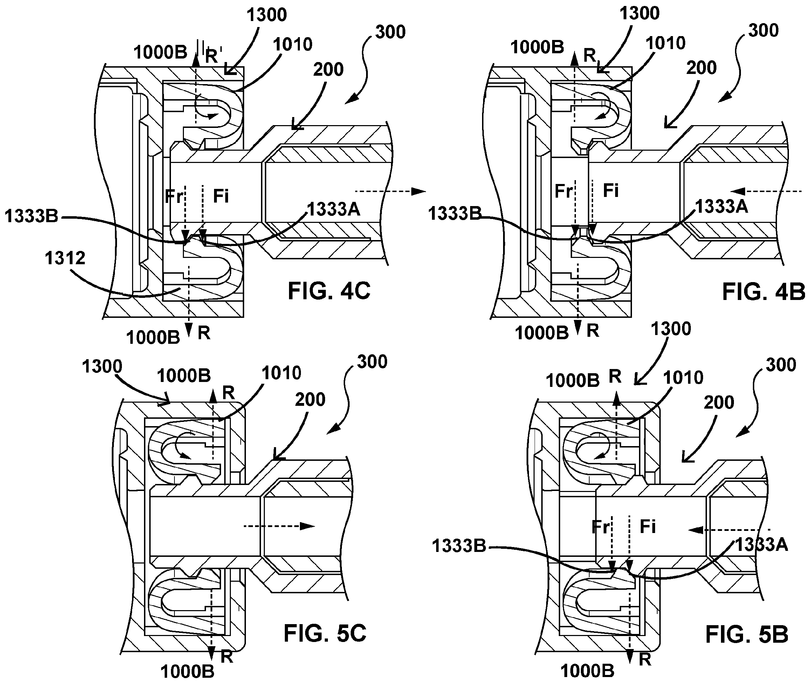

Figures 4B and 4C, respectively, are cross-sectional views showing a device that is being inserted through an embodiment of a coupling mechanism, and the device being removed from the coupling mechanism;
Figures 5B and 5C, respectively, are cross-sectional views showing a device being inserted through an alternate embodiment of a coupling mechanism, and the device being removed from the coupling mechanism;

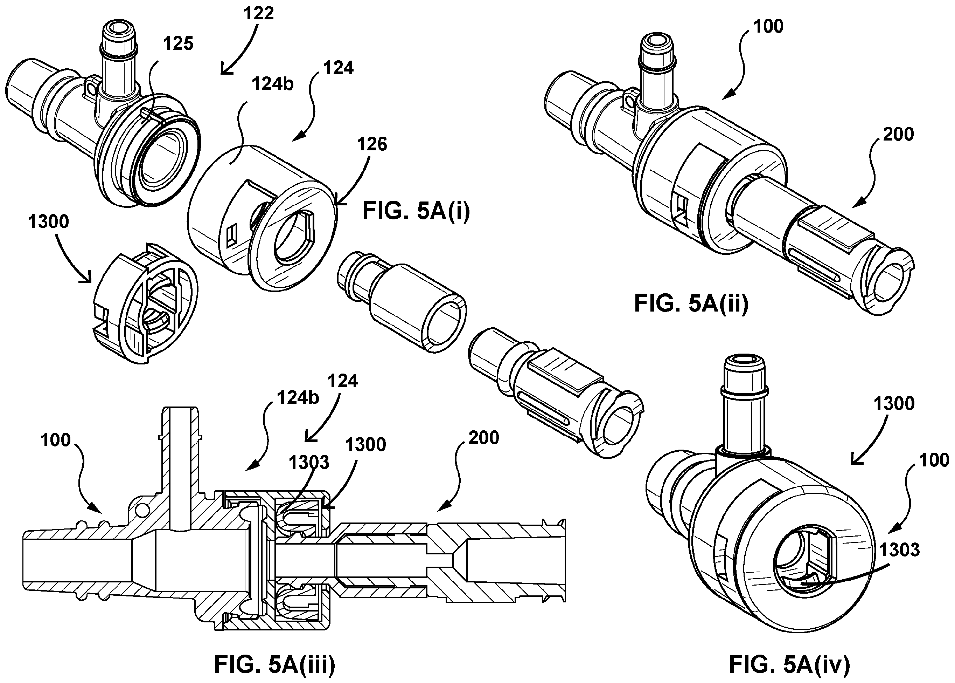

Figures 5A(i), 5A(ii) and 5A(iii) are, respectively, a perspective view, an exploded view, and a cross section through a hub containing an embodiment of a coupling mechanism of the present invention, as well as a device inserted through the coupling mechanism;
Figure 5A(iv) is a perspective view of an embodiment of a hub and coupling mechanism of the present invention;

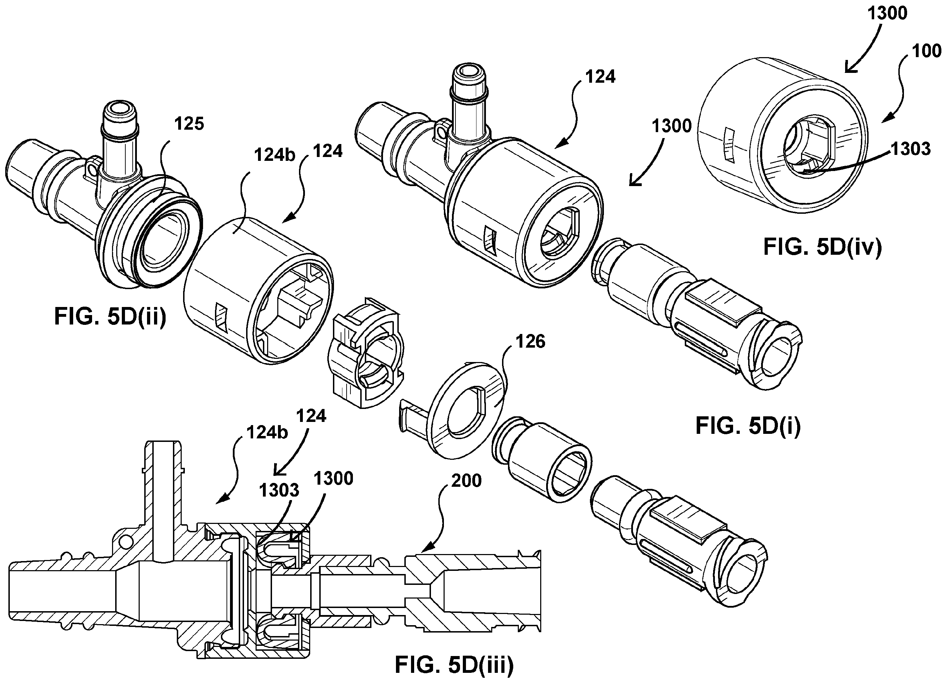

Figures 5D(i), 5D(ii) and 5D(iii) are, respectively, an exploded view, a perspective view and a cross section through a hub containing an embodiment of a coupling mechanism of the present invention;
Figure 5D(iv) is a perspective view of an embodiment of a hub and coupling mechanism of the present invention;

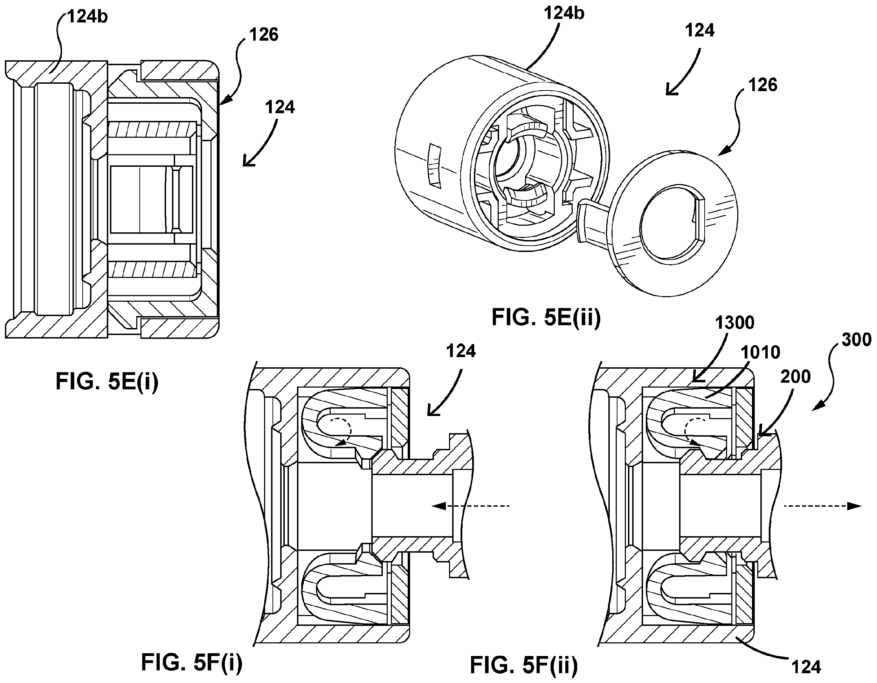

Figures 5E(i) and SE(ii) show, respectively, cross-sectional and perspective views of an embodiment of a housing and a coupling mechanism of the present invention;
Figures 5F(i) and 5F(ii) are cross-sectional illustrations of a device being inserted through an embodiment of a coupling mechanism of the present invention and being removed therefrom;
Figures 5A(i), 5A(ii) and 5A(iii) are, respectively, a perspective view, an exploded view, and a cross section through a hub containing an embodiment of a coupling mechanism of the present invention, as well as a device inserted through the coupling mechanism;
Figure 5A(iv) is a perspective view of an embodiment of a hub and coupling mechanism of the present invention;
Figures A(i) and 6A(ivi) are, respectively, perspective views of a device partially and fully inserted into a hub comprising an embodiment of a coupling mechanism of the present invention;

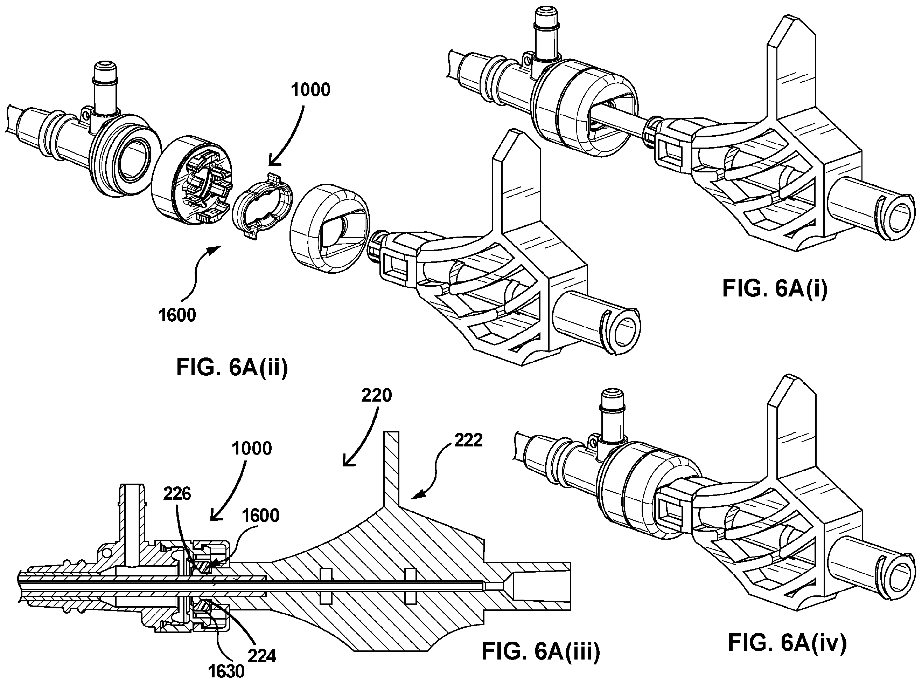

Figures 6A(ii) and 6A(iii) are, respectively, an exploded view and a cross section through a hub containing an embodiment of a coupling mechanism of the present invention, as well as a device inserted through the coupling mechanism;

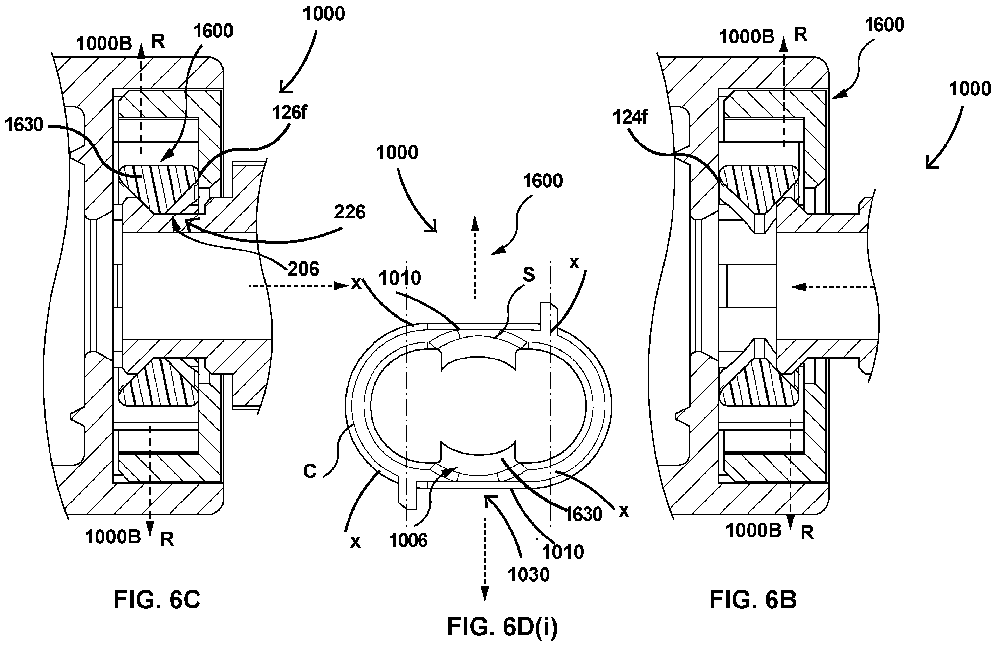

Figures 6B and 6C are, respectively, cross sectional views of a device inserted through an alternate embodiment of a coupling mechanism of the present invention and being removed therefrom;

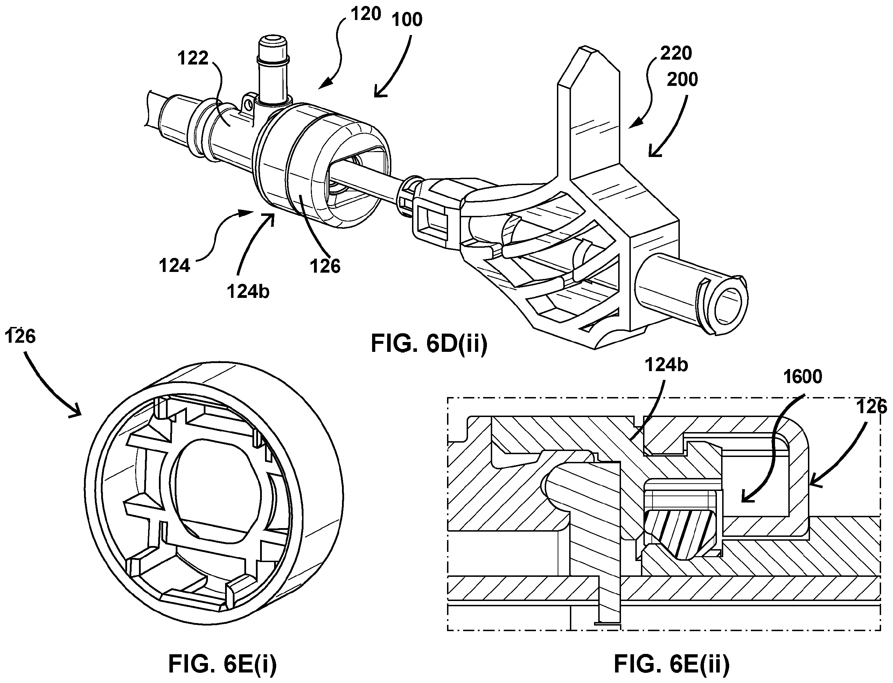

Figure 6D(i) is a side view of an alternative embodiment of a coupling mechanism of the present invention;
Figure 6D(ii) shows a perspective view of a device partially inserted through an embodiment of a coupling mechanism of the present invention;
Figure 6E(i) shows a side view of an embodiment of a cap of a hub;
Figure 6E(ii) is a partial cross-sectional view of an alternate embodiment of a coupling mechanism located within a hub;

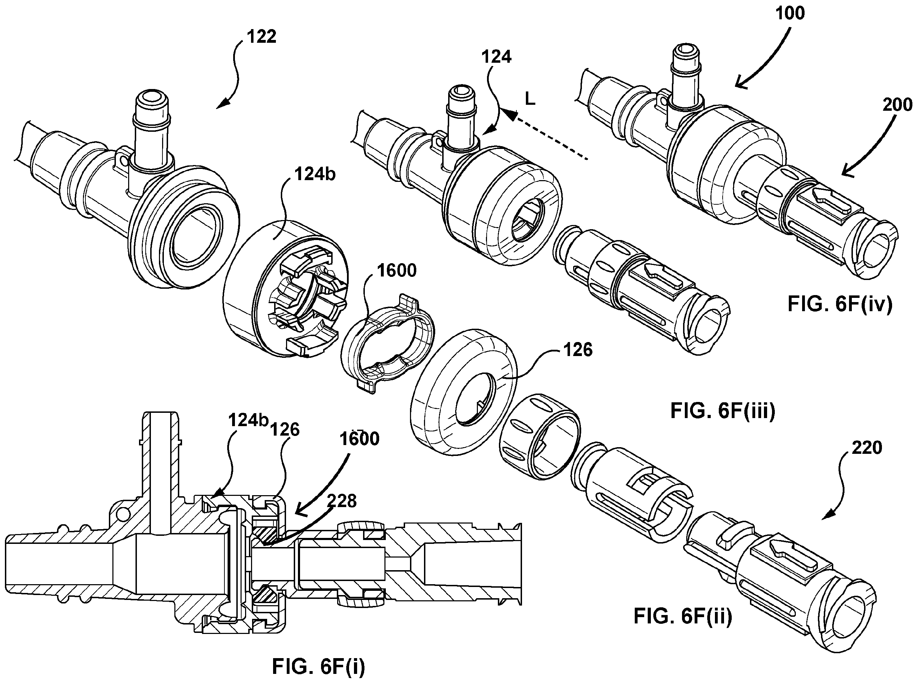

Figures 6F(i) and 6F(ii) are, respectively, a cross section through, and an exploded view of, a hub containing an embodiment of a coupling mechanism of the present invention, as well as a device inserted through the coupling mechanism;
Figures 6F(iii) and 6F(iv) are, respectively, perspective views of a device being inserted into a hub and being removed therefrom comprising an embodiment of a coupling mechanism of the present invention;

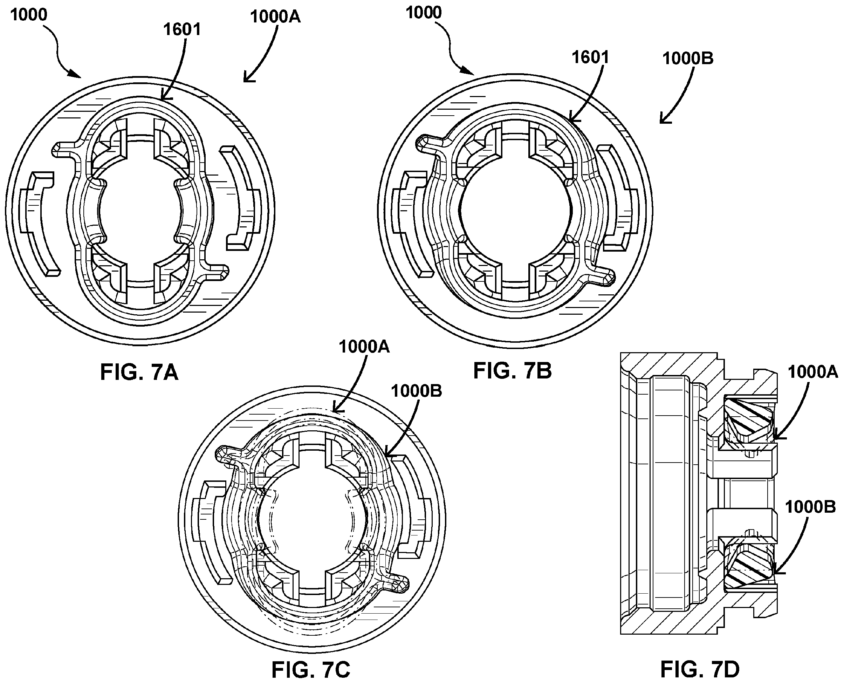

Figures 7A, 7B and 7C illustrate top views of a coupling member in accordance with an embodiment of the present invention in its different states;
Figure 7D illustrates a side cross-sectional view of a coupling member in accordance with an embodiment of the present invention in its different states;

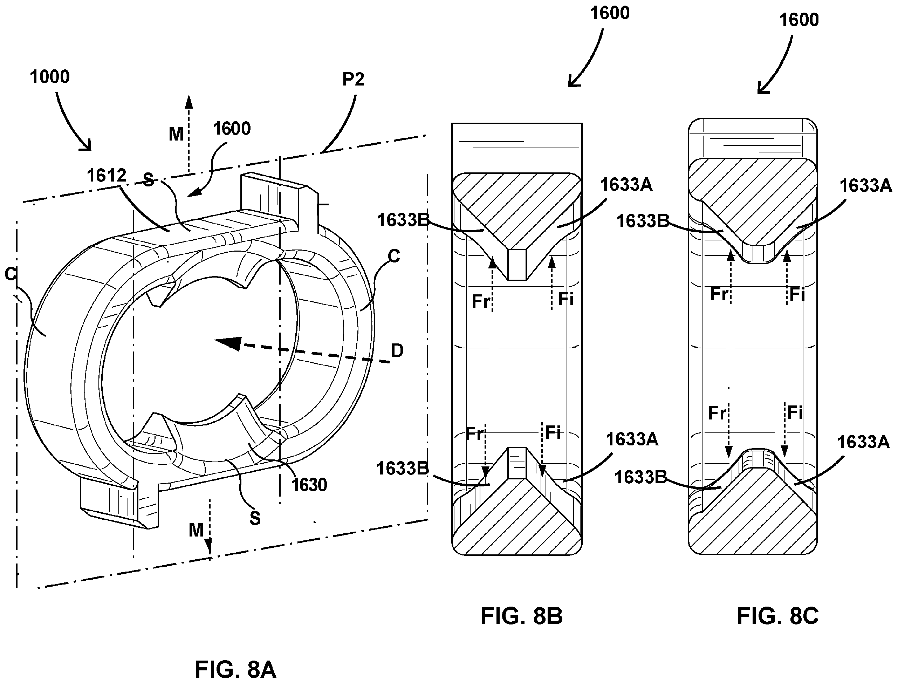

Figure 8A is a perspective view of a coupling member in accordance with an alternative embodiment of the present invention;
Figure 8A is a perspective view of a coupling member in accordance with an alternative embodiment of the present invention;
Figures 8B and 8C are different cross-sectional views of the coupling member taken along the mid-point of the coupling member of Fig. 8A, in accordance with an alternative embodiments of the present invention.

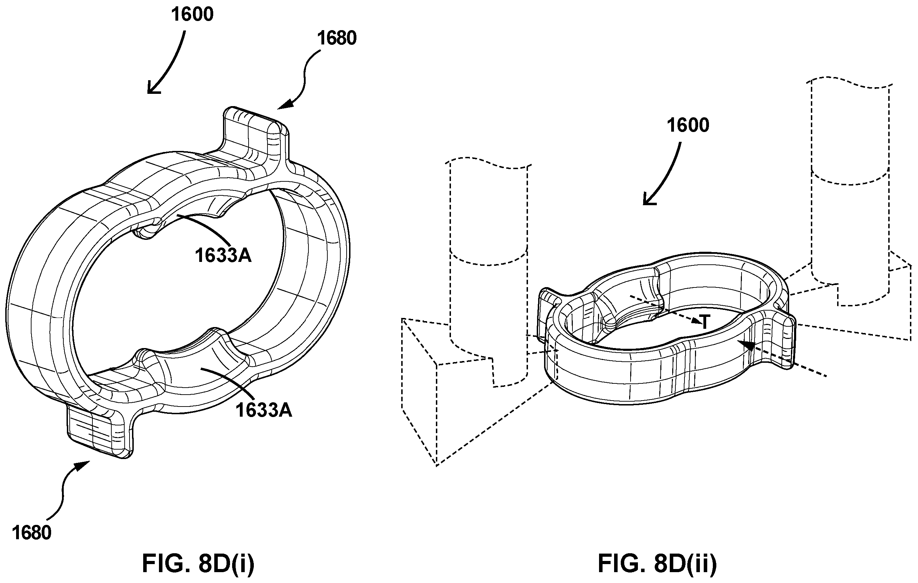

Figures 8D(i) and 8D(ii) are a perspective view of a coupling member in accordance with an alternative embodiment of the present invention;

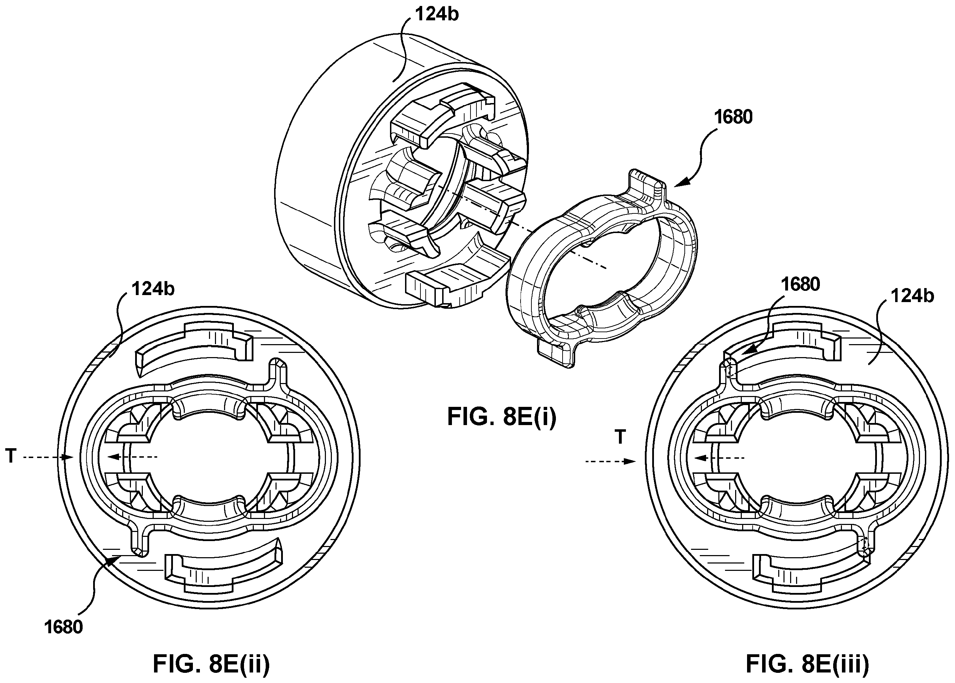

Figures 8E(i), and 8E(iii) are a top view of a coupling member in accordance with an embodiment of the present invention and a portion of the housing for retaining the same;
Figure 8E(ii) is a perspective view of a coupling member in accordance with an alternative embodiment of the present invention as well as a portion of the housing for retaining the same;
Figures F(i), and 8F(iii) are Figures 6B and 6C are, respectively, cross sectional views of a device inserted through an alternate embodiment of a coupling mechanism of the present invention and being removed therefrom;

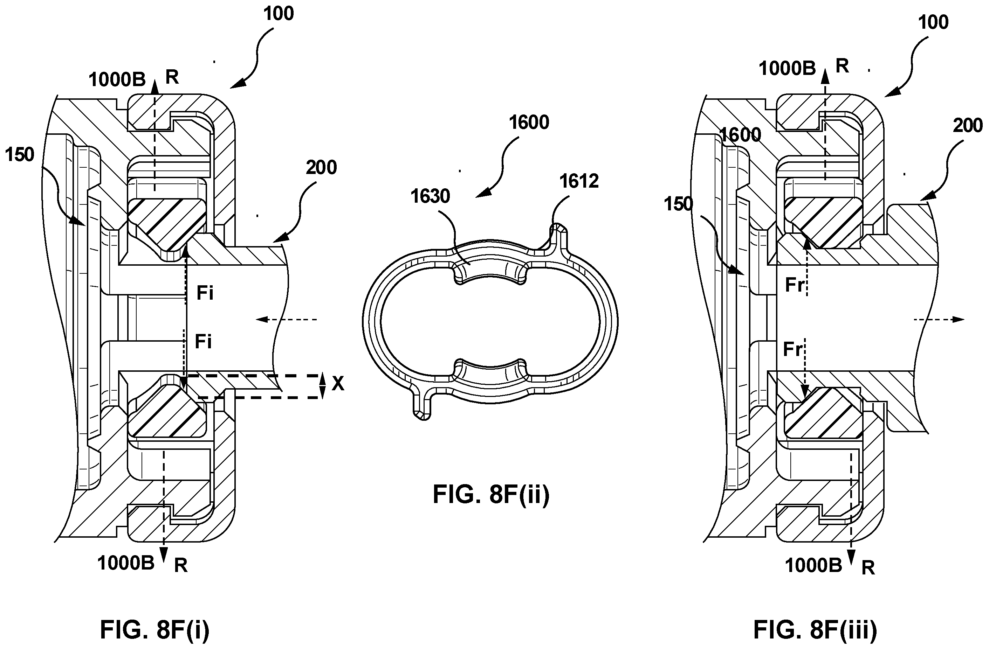

Figure 8F(ii) is a perspective view of a coupling member in accordance with an alternative embodiment of the present invention as well as a portion of the housing for retaining the same;

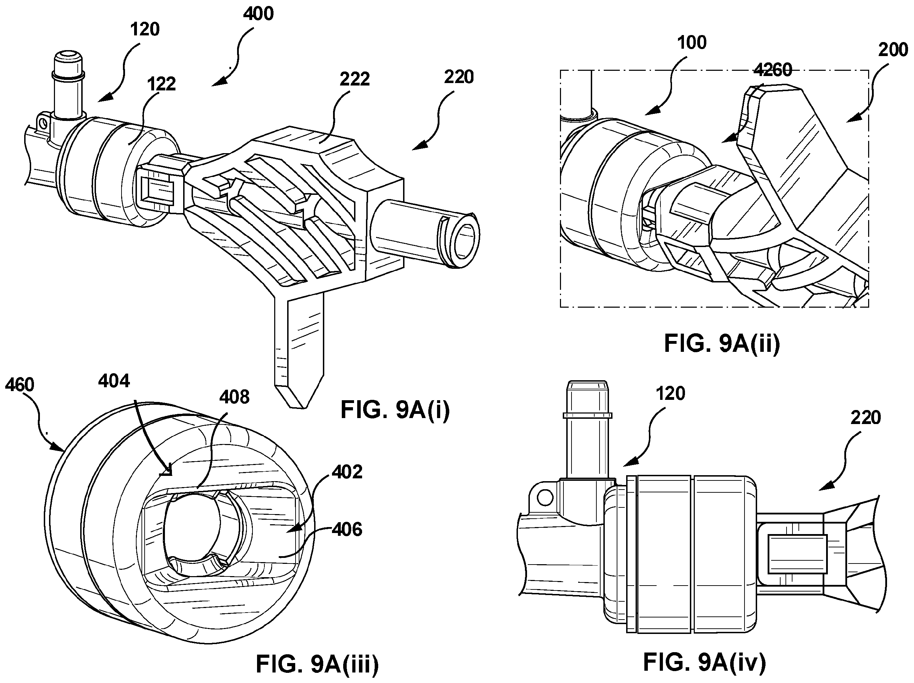

Figures 9A(i), 9A(ii), 9A(iii) and 9A(iv) are perspective views of components of a rotational locking mechanism comprising a first mating member and a second mating member;

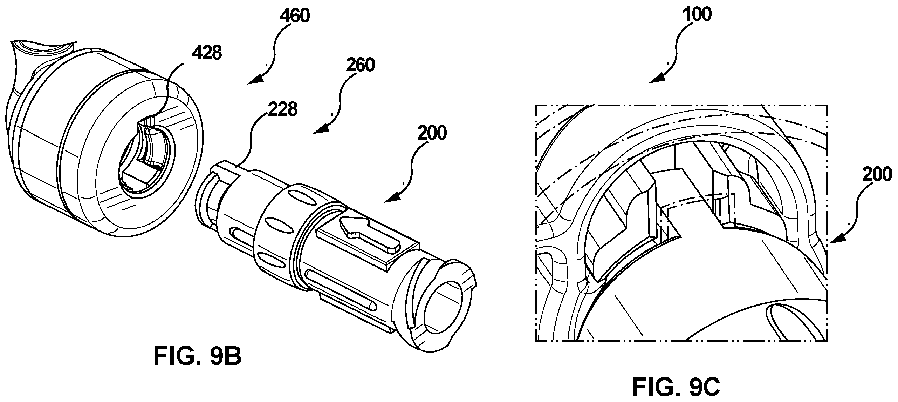

Figures 9B and 9C are perspective views of components of a rotational locking assembly comprising a first mating member and a second mating member and engagement there-between, in accordance with an alternative embodiment of the present invention;

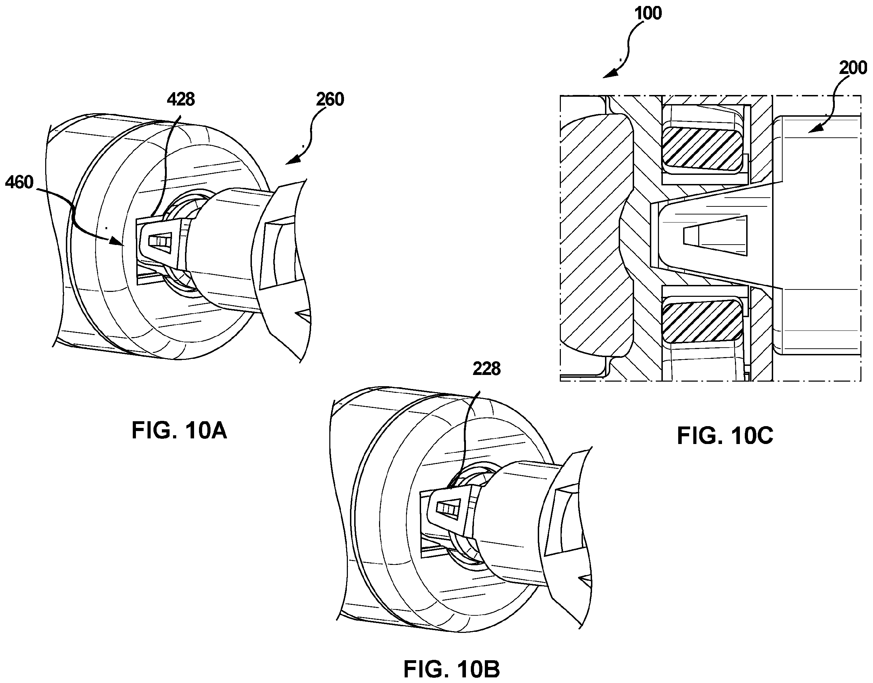

Figures 10A and 10B are perspective views of components of a rotational locking assembly comprising a first mating member and a second mating member and engagement there-between, in accordance with an alternative embodiment of the present invention;
Figures 10C is a side cross-sectional view of the rotational locking assembly of Fig. 10A, in accordance with an alternative embodiment of the present invention;

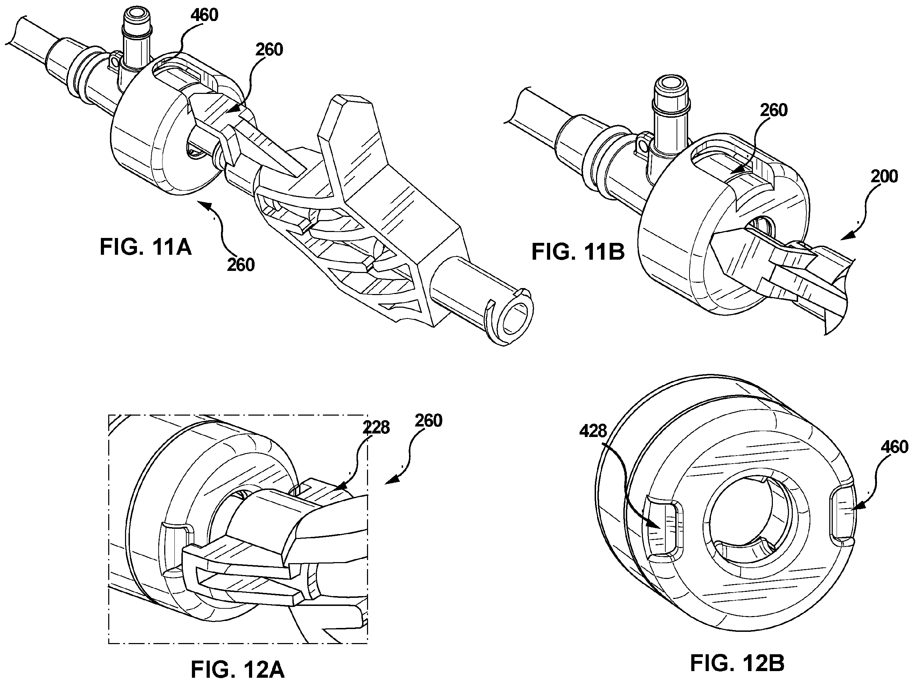

Figures 11A and 11B are perspective views of components of a rotational locking assembly comprising a first mating member and a second mating member and engagement there-between, in accordance with an alternative embodiment of the present invention;
Figures 12A and 12B are perspective views of components of a rotational locking assembly comprising a first mating member and a second mating member and engagement there-between, in accordance with an alternative embodiment of the present invention;

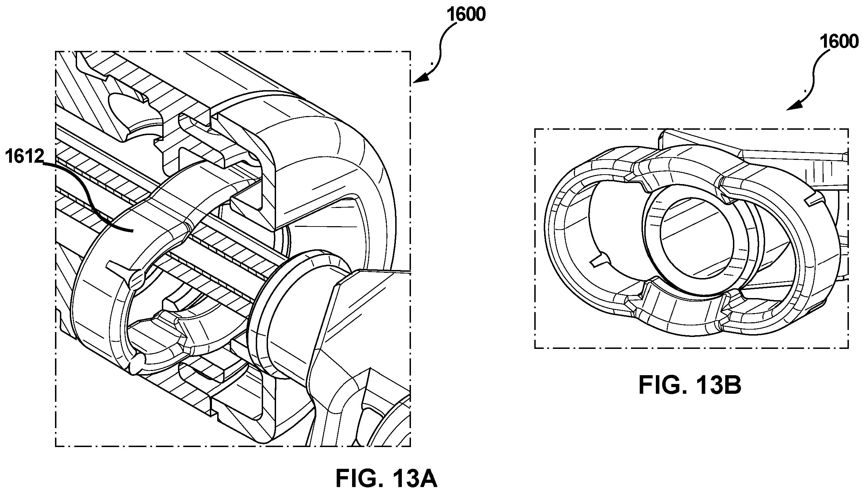

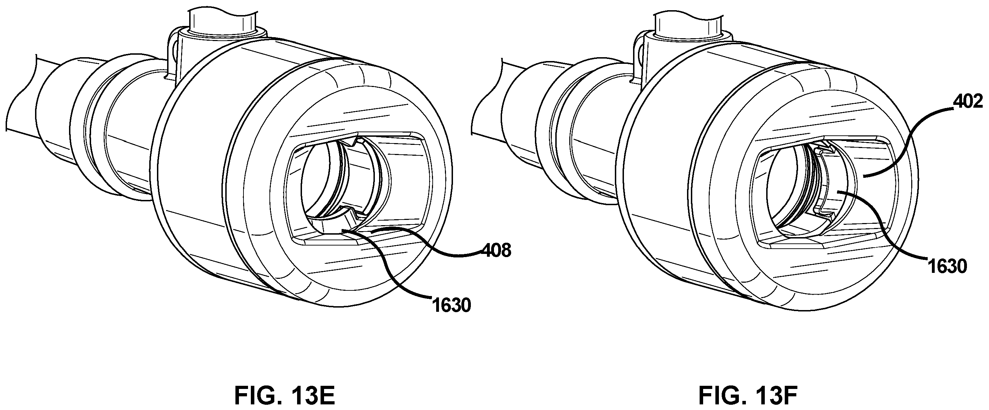

Figures 13A, 13B and 13E are perspective views of a coupling mechanism comprising a coupling member, in accordance with an alternative embodiment of the present invention;

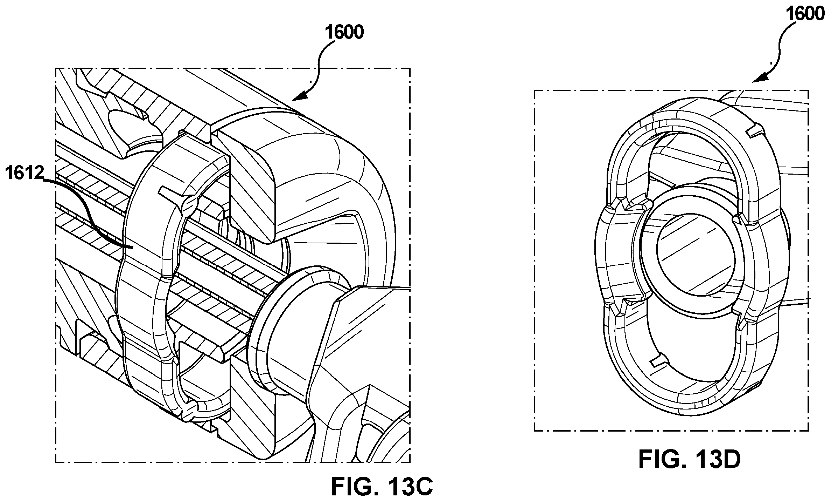

Figures 13C, 13D and 13F are perspective views of a coupling mechanism comprising a coupling member, in accordance with an alternative embodiment of the present invention;

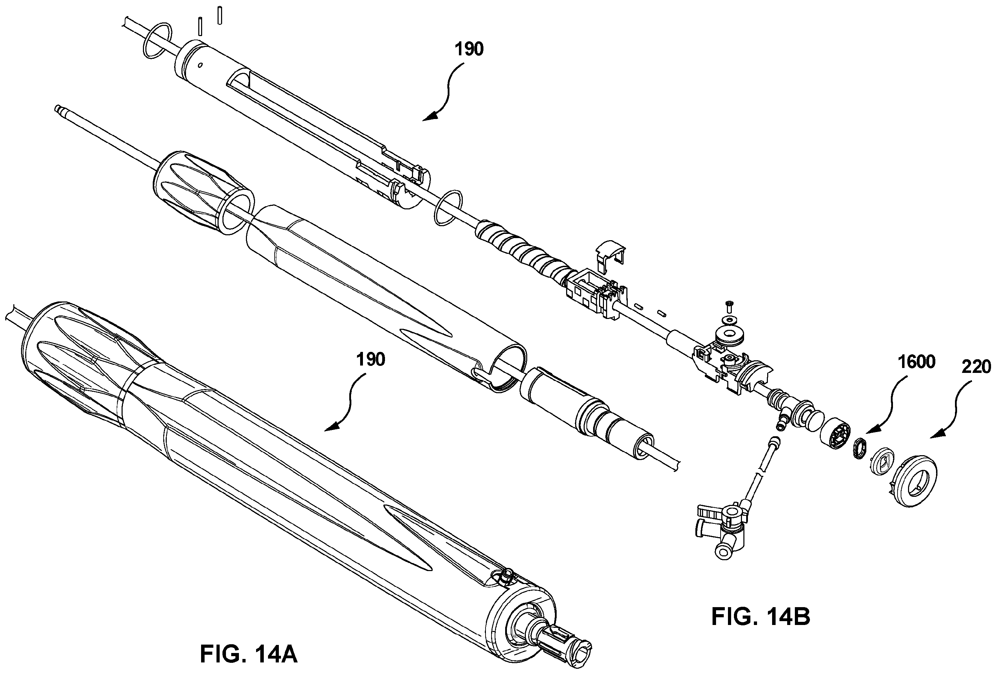

Figures 14A and 14B are, respectively, an exploded view and a perspective view of an alternate embodiment of the first mating member containing an embodiment of a coupling mechanism of the present invention, as well as a device inserted through the coupling mechanism;

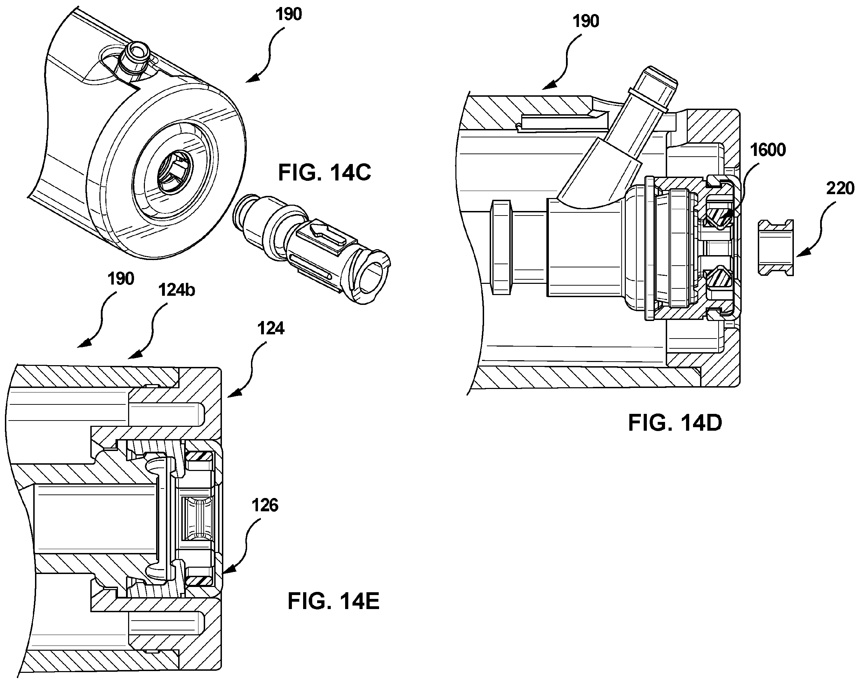

Figures 14C is a perspective view of an alternate embodiment of the first mating member comprising an embodiment of a coupling mechanism of the present invention, in accordance with an embodiment of the present invention; and
Figures 14D and 14E are cross sectional views taken along different sections of the second mating member shown in Figs. 14C, that comprises an embodiment of a coupling mechanism of the present invention, as well as a device inserted through the coupling mechanism, in accordance with an embodiment of the present invention.

### DETAILED DESCRIPTION

With specific reference now to the drawings in detail, it is stressed that the particulars shown are by way of example and for purposes of illustrative discussion of certain embodiments of the present invention only. Before explaining at least one embodiment of the invention in detail, it is to be understood that the invention is not limited in its application to the details of construction and the arrangement of the components set forth in the following description or illustrated in the drawings. The invention is capable of other embodiments or of being practiced or carried out in various ways. Also, it is to be understood that the phraseology and terminology employed herein is for the purpose of description and should not be regarded as limiting.

In order to carry out certain medical procedures such as trans-septal procedures, it is necessary to gain access to the heart specifically to the left atrium of the heart. Access may be obtained to the heart from vasculature using one or more medical devices such as an introducer or sheath. In order to gain access, a superior approach may be used (by gaining access to the heart for example from the jugular vein through the superior vena cava), or alternatively access may be obtained from the femoral or inferior approach (by gaining access to the heart from the femoral vein through the inferior vena cava). Once access is obtained into the left atrium, one or more additional devices may be advanced through the introducer or sheath to carry out a part of the procedure. For example, in order to carry out trans-septal puncture, a puncture device is advanced through vasculature (for example through the sheath and/or through a dilator positioned within the sheath) in order to puncture through tissue (creating a puncture site) for example across a septum of the heart to gain access from the left atrium into the right atrium of the heart. Additionally, as mentioned above, a dilator may be used during a part of the procedure. For example, in combination with the puncture device, a dilator may be advanced to the puncture site to dilate the puncture site to enable additional medical devices to be advanced through the puncture site into the left side of heart. In such procedures, the dilator may be locked or coupled to the introducer or sheath using a locking mechanism during a portion of the procedure allowing the two devices to be advanced concurrently and/or to enable two of the devices to be coupled together thereafter after they are positioned relative to one another. The locking mechanism additionally enables decoupling of the two devices to enable one of the devices to be advanced independently during a part of the procedure.

In conventional systems locking mechanisms are provided that connect two devices at the proximal portions thereof for example along the hub portions, which ensures that the distal portions remain fixed in the desired or correct position while the user guides the sheath and dilator inside the patient anatomy. In some such examples, the direction of the sheath curve is indicated by the side port on the sheath hub and is controlled by rotating the sheath hub. The locking mechanism couples the dilator to the sheath allowing the sheath and dilator to be advanced and/or rotated together.

For example, the once the devices are positioned at the septum prior to puncture for example, the dilator snaps into the sheath hub to connect the two devices. This is done by the user on the proximal end which is the user interface. By connecting the two devices proximally, it ensures the distal portions remain fixed in the correct position while the user guides the sheath and dilator in the patient anatomy.

Certain limitations may be associated with the use of medical devices such as introducers or sheaths and dilators that employ conventional locking mechanisms such as snaps. The limitations of the existing locking mechanisms on these devices may include one or more of the following. The existing locking mechanisms have: snaps which degrade with use, provide insufficient retention force, provide insufficient tactile feedback, and/or generate debris. Additionally, the locking mechanism may not provide desired insertion force and/or removal force.

In some such examples, it may be too difficult to or require too much force to snap or connect the two devices together making it difficult for the user to couple the two devices, and/or may require too much force to unsnap or disconnect the two devices which could lead to loss of positioning of the devices. Conversely, it may be too easy to or require very little force to snap or connect the two devices together and/or may require very little force to unsnap or disconnect the two devices, which may not provide sufficient retention force and may lead to undesired and/or unintentional decoupling of the devices.

Some such conventional medical devices such as sheaths and dilators that require the use of rigid snaps have snap fits that have a rigid ring or bump on the dilator hub that must press into a mating feature on the sheath hub. These rigid snap mechanisms may have one or more of the above noted problems which may include one or more of: (1) degrading with use, (2) providing insufficient retention force, (3) providing insufficient tactile feedback, or generating debris, or (4) not providing a uniform force for locking and providing a uniform force for repeated uses.

Additionally, other problems associated with conventional snap mechanisms is that they require the use of plastic deformation of material, where the snaps are designed to and require that they deform plastically to enable coupling or locking of the two components. Such mechanisms rely on degradation of the snap component to enable locking. As such the initial insertion force value to enable coupling or locking for the first time may be high whereas with multiple uses, including starting from the second or third use for instance, the insertion force required to insert the dilator hub into the sheath hub may decline rapidly. As such the user may need to use a very high force to snap the two devices initially and the user may additionally get a different feel with multiple uses, providing a varying and inconsistent user experience. The prior art snaps require the user to use a varying amount of insertion force to couple the sheath and dilator hubs together to snap the two hubs together. This provides the user with varying feedback on the force that is required in order to snap the two components together.

Such prior art systems rely on locking mechanisms that utilize plastic deformation to provide coupling or locking using a press fit between rigid rings or bumps or tabs on the two medical devices that are being coupled. As such conventional systems rely on plastic deformation and not able to retain their shape during multiple uses and can be deformed or degraded over time.

Furthermore, existing snap mechanism may not adequately couple two devices to allow them to be rotated together and/or allowing the curves of the two devices to be aligned to provide directionality.

As such, there is a need to provide a coupling mechanism that allows two devices to be coupled together, while providing a relatively uniform insertion force and/or removal force in order to couple the two devices and for repeated uses (for example to snap the two devices together). There is additionally a need in the prior art to provide a mechanism that provides one or more advantages of (1) not degrading with use or multiple uses, (2) providing sufficient retention force to enable coupling, (3) providing insufficient tactile feedback (4) generating debris or (5) not providing a uniform force for locking for repeated uses (6) providing sufficient force for locking that is sufficient for retention but the force is not too high that is makes it difficult for the user to snap the two devices together.

Still furthermore, there is additionally a need to provide a mechanism that couples two devices so they can be rotated together and provide directionality by allowing the curves of the two devices to be aligned.

The inventors of the present invention have discovered novel locking mechanism and systems and methods that use the same in an attempt to overcome the limitations associated with prior art locking mechanism and systems.

In one broad aspect, the present inventors have discovered systems and methods that provide a novel locking mechanism comprising a snap fit design that enables flexible coupling between two medical devices such as a sheath and/or dilator. The systems and methods of the present invention attempt to overcome limitations associated with conventional locking mechanisms and systems that utilize rigid snap mechanisms.

Inventors of the present invention have developed a novel locking or coupling mechanism for coupling or locking two medical devices and a system that uses the same. The novel mechanism as provided herein is a releasable coupling or locking mechanism that provides a coupling member or element such as a flexible coupling member that provides a flexible coupling at the interface between the two devices, for example at the interface between the proximal portion of the two devices. In other words in some embodiments of the present invention, a flexible coupling member is provided that allows first and second mating members of a coupling system or arrangement to be releasably coupled to one another.

More specifically, in some embodiments the releasable coupling or locking mechanism defines a flexible coupling mechanism that comprises a flexible snap fit mechanism at the interface between the two devices where the flexible snap fit mechanism provides: a flexible snap member or component (or in other words a flexible coupling member or component) [either independent or attached] at the interface between the proximal portions or in other words mating portions of the two devices.

In other embodiments the releasable coupling or locking mechanism defines a flexible coupling mechanism that comprises flexible a coupling member or component comprising a snap fit mechanism at the interface between the two devices where the coupling mechanism provides: a member or element comprising a moveable and/or a flexible locking member or component (or in other words a moveable and/or a flexible coupling member or component) [either independent or attached] at the interface between the proximal portions of the two devices (or first and second mating members).

In some such embodiments, a coupling element comprising a flexible and/or a moveable member or component such as a flexible snap member or component is used at the interface between the two hubs of the two devices such as a sheath hub and the dilator hub. For example, the flexible snap member or component is provided within the sheath hub, where the flexible snap member or component interacts with the dilator hub to couple it to the sheath hub where the snap member or component itself is flexible and is capable of providing elastic deformation.

Some such embodiments of the present invention provide a flexible locking mechanism comprising a coupling element comprising a flexible locking member or component. In one particular example the flexible locking mechanism comprises defines a flexible snap fit mechanism that relies on elastic deformation to enable releasable coupling between the sheath hub and the dilator hub. More specifically, the flexible snap fit mechanism comprise a flexible locking member or component such as a flexible snap member or component (or interface or assembly) that flexes. As such, the flexible snap fit retains its shape during multiple uses and is not substantially deformed or degraded as the user is not plastically deforming the material as the dilator hub is advanced into the sheath hub. As an additional advantage in some embodiments the flexible snap member or component allows the snap-in force and the snap-out force to be varied.

In some such examples, the coupling element comprising the flexible snap member or component is provided independent from the device hub such as the sheath hub, or in other embodiments the snap member or component is attached to the device hub such as the sheath hub.

In some embodiments, the snap member or component comprises a resilient snap member or component that is provided within the sheath hub that is where the snap member or component [for example comprises a flexible body or body portion] where the body portion and thus the snap member or component itself is flexible and is capable of providing elastic deformation to allow the resilient snap member or component to move out [for example radially] to allow a portion of the dilator hub to pass and move back in [for example against a portion of the dilator hub or into a groove of the dilator hub] to retain it by releasably coupling it to the sheath hub. Then when the dilator is pulled back the resilient snap member or component can move out again to allow the dilator to be removed by disengaging the locking mechanism.

In some such embodiments, the body portion of the resilient snap member or component (and the resilient snap member or component) is configured to change its shape and/or move out of the way upon advancement of the dilator hub within the sheath hub, to allow a portion of the dilator hub to pass, such as a rigid bump or ring on the dilator hub, the body portion of the resilient snap member (and thus the resilient snap member or component) is then configured to move back into its original shape and/or position [for example into a groove of the dilator] in order to retain the dilator hub within the sheath hub to releasably lock the sheath and dilator hubs. Then when the dilator and thus the dilator hub is pulled back the body portion of the resilient snap member or component (and thus the resilient snap member or component) is configured to change its shape and/or move out again to allow the dilator hub to be removed, allowing the body portion of the snap member or component (and thus the snap member or component) to go back to its original shape and/or position.

In a specific example, the snap member or component comprises a resilient snap ring or band that is for example oval shaped that is configured to change its shape to a circular snap ring or band and move out [for example radially] to allow a raised portion of the dilator hub (such as a bump or a ring) to pass past the resilient ring or band (specifically past one or more snaps positioned along the body portion such as the resilient ring or band). The circular resilient ring or band is then configured to change or move back into its original oval shape (an oval snap ring or band) and thus its original position (allowing it to be positioned within a groove of the dilator hub). Specifically, allowing the one or more snaps along the oval resilient snap ring or band to move into a groove of the dilator hub, in order to retain it within the sheath hub to releasably couple the dilator hub to the sheath hub using the flexible locking mechanism. Then when the dilator hub is pulled back the oval snap member or component can move out again to allow the dilator hub to be removed and the snap member or component is configured go back to its original oval shape and position within the sheath hub.

In some such examples, the snaps along with the resilient snap ring or band are flexible and are also capable of elastic deformation allowing the assembly of the snap ring or band and the snaps to flex out of the way (for example radially) upon insertion of the dilator and to move back to its original shape and/or place to couple the sheath hub to the dilator hub. In some such examples, the snaps comprise flexible tabs that along with the snap ring or band are flexible, where the assembly of the snap ring or band and flexible tabs flexes and move out of the way upon advancement of the dilator hub and then moves back to its original shape and/or place to couple the sheath hub to the dilator hub. In some such embodiments the flexible snap member or component is moveable into a groove of the dilator hub (for example as it moves radially back into its original position) and sits within the groove of the dilator hub and may not hug the wall of the groove. In other embodiments, the flexible snap member or component is moveable into and sits within the groove of the dilator hub and may additionally hug the wall of the groove.

In some embodiments of the present invention, a locking mechanism is provides that provides a novel solution for releasably coupling two medical devices, where a moveable coupling member or component or portion is provided within the sheath hub, where the moveable coupling component or portion [attached or unattached] is translatable or moveable out of the way of the dilator hub wider portion (for example radially) as the dilator hub is advanced through and past the opening of the sheath hub. In some such examples the moveable coupling member or component comprises a moveable locking member or component. In some such examples the moveable coupling member or component is a moveable snap member or component. Then once the dilator hub wider portion is past the moveable component or portion - the moveable coupling component or portion is then moveable, for example radially, into the groove of the dilator hub [or in other words dilator hub groove portion] - and the (component or member which in some examples comprises a flexible snap) is thus positioned in the dilator groove [and thus coupled thereto] and as such the effective diameter of the dilator hub is now wider than the sheath hub opening preventing the dilator hub to be readily retracted from the opening in the absence of force.

In other words, once the dilator hub has advanced past the moveable member or component into the sheath hub, the moveable member such as a flexible snap member or component moves radially back into its original shape or configuration enabling the flexible snap member or component [such as a resilient snap ring or band] to fall into place within the dilator hub groove portion which creates a larger effective outer diameter of the dilator hub than the sheath hub opening creating an interference fit for releasably coupling the dilator hub to the sheath hub and preventing the dilator hub to be decoupled from the sheath hub in the absence of force. In other words, an interference fit is created between the moveable coupling member that is seated within the dilator hub groove, and the dilator hub groove. And the moveable coupling member may be seated loosely within the dilator hub groove. This arrangement of the coupling member within the dilator hub groove enables translational locking the sheath and dilator hubs and prevents the dilator hub from being advanced further into the sheath hub and additionally from being retracted as well, as the effective diameter of the dilator hub is greater than the opening of the sheath hub. In other words, the interaction between the moveable coupling member and the distal inner wall of the sheath hub housing (that defines the opening or space within which the moveable coupling member is seated) prevents the sheath hub from being retracted proximally in the absence of force. Similar interaction between the proximal inner wall of the sheath hub housing and the moveable coupling member prevent the dilator hub from being advanced further proximally once it is inserted into sheath hub. Thus the moveable coupling member of the present invention enables translational locking of the sheath and dilator hubs.

As such, in some embodiments, the moveable component functions to block the movement of the dilator hub out of the sheath hub and functions to create an interference fit to prevent movement of the dilator hub in the absence of force. This provides a translation locking mechanism where the dilator hub is not able to advance further into the sheath hub and additionally prevents the dilator from being retracted/disengaged or removed from the dilator hub in the absence of force.

In some such embodiments, a portion of the moveable and/or flexible member or component may be flexible or resilient or the moveable and/or flexible member or component may be partially flexible or resilient. In some such examples, the moveable and/or flexible member or component comprises a spring biased member.

In other embodiments, the moveable member or component may not be flexible or resilient, but is functional to move into the groove of the dilator to block the movement of the dilator hub out of the sheath hub, in the absence of force for instance. In one such example, the moveable locking member or component may comprise one or more loose pieces within the sheath hub that move (for example radially) out of the way when the dilator hub (specifically the dilator hub wider portion) is advanced into the sheath hub and once the dilator hub is advanced further such that the dilator hub groove portion is positioned at the axial location of the moveable locking member or component allowing the loose pieces to fall into place within the dilator hub groove portion which may create a larger effective outer dilator than the sheath hub opening creating an interference fit releasably coupling the dilator hub to the sheath hub and preventing the dilator hub to be decoupled from the sheath hub in the absence of force. However, once a sufficient force is applied the dilator hub may be decoupled from the sheath hub by allowing the loose pieces to move radially out from the groove upon application of a pulling force on the dilator.

As such in some embodiments of the present invention a locking mechanism is provided where the dilator has a smaller effective diameter going into the sheath hub and once in - it has a larger effective diameter by coupling to the member or component (such as a snap member or component) of/in the sheath hub, preventing it from exiting in the absence of force [where force is used for decoupling the component from the dilator hub]. The moveable member or component such as a moveable locking member or component in the sheath hub that creates an interference fit/or interacts with the incoming dilator hub preventing it from exiting - for example by creating a larger effective diameter on the dilator hub. In some such examples, the moveable member or component of the locking mechanism within the sheath hub is a flexible component. In other examples the member or moveable component of the locking component within the sheath hub is a translating or moveable component. In other embodiments, the locking member or component of the locking mechanism may be flexible and/or moveable, and functions to block the movement of the dilator hub out of the sheath hub in the absence of force. In some such embodiments, the moveable locking member or component comprises a flexible locking member or component, for example comprising a resilient material as noted above.

Some such embodiments of the present invention provide an audible click when the flexible or moveable snap member or component of the snap fit mechanism co-operatively or interacts with the dilator hub to enable engagement or coupling between the dilator hub and the sheath hub, and functions to provide the user with an audible indication - such as a clicking sound, indicating that the two hubs have been coupled or effectively locked together. In some such embodiments as noted above, the snap member of component may comprise one or more snaps positioned along a portion thereof such as a body portion, such as a resilient ring or band. In some such examples, the snaps may function to create audible sound or indictor such as a clicking sound to indicate that the two hub portions have been coupled or alternatively to indicate when the two hub portions are then disengaged.

As such embodiments of the present invention provide locking mechanisms that overcome one or more disadvantages of prior art locking mechanisms such as existing snap fits that have a rigid ring or bump on the dilator hub that must press into a mating feature on the sheath hub, as such embodiments of the present invention avoid the disadvantages of locking mechanisms that involve plastic deformation of the bumps/detents on the sheath/dilator hubs, and as such locking mechanism as presently disclosed herein can solve one or more problems of prior art locking mechanisms such as degrading with use, providing insufficient retention force, providing insufficient tactile feedback, and/or generating debris.

### A releasable coupling mechanism

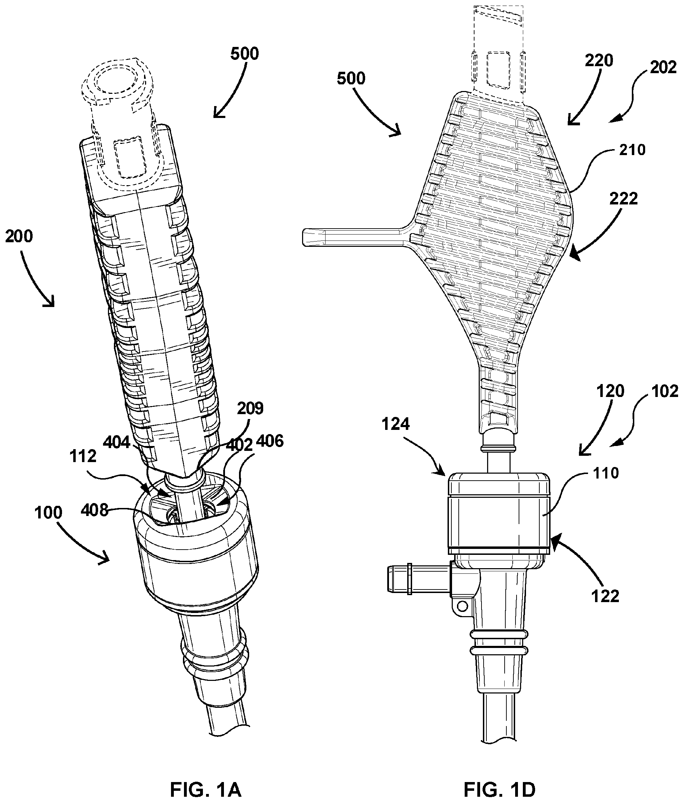

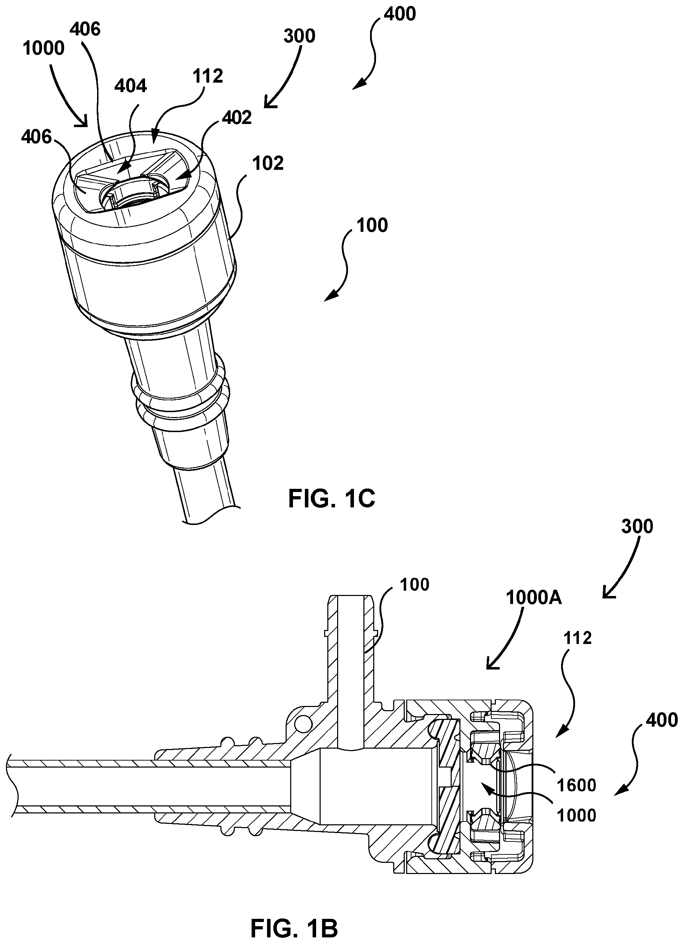

In some embodiments of the present invention as shown in Fig. 1A, a releasable coupling mechanism 300 is provided for releasbly coupling two members such as a first mating member 100 and a second mating member 200. As additionally shown in Figs. 1B and 1C, the releasable coupling mechanism 300 comprises a coupling member or component 1000 associated with a first mating member 100 for releasably coupling or engaging a second mating member 200 to the first mating member 100, where the second mating member 200 is receivable by the first mating member 100, for example through an opening 112 thereof. In some such examples, the coupling member 1000 is positioned inside or held within the first mating member 100, as shown in Fig. 1B.

In some such embodiments, the coupling member 1000 has a first state 1000A and a second state 1000B [shown in Figs. 7A-7B] and is moveable there-between to enable the second mating member 200 to be coupled to the first mating member 100. Specifically, the coupling member 1000 is moveable from the first state 1000A into a second state 1000B upon insertion of the second mating member 200 into the first mating member 100 to allow passage of the second mating member 200 and the coupling member 1000 is moveable thereafter into the first state 1000A to couple the second mating member 200 to the first mating member 100, discussed further herein below.

### Releasable Coupling Assembly

In accordance with some embodiments of the present invention releasable coupling assembly 400 is provided that comprises the first mating member 100 and a coupling mechanism 300 as described herein above, as shown in Figs. 1A, 1B and 1C. More specifically, the releasable coupling assembly 400 comprises, the first mating member 100 and a coupling mechanism 300 comprising a coupling member 1000 associated with the first mating member 100 for releasably coupling a second mating member 200 receivable by the first mating member 100, to the first mating member 100. As noted herein above, the coupling member 1000 has a first state (or configuration) 1000A and a second state (or configuration) 1000B as shown in Figs. 7A and 7B, as discussed further herein below. As noted herein, the coupling member 1000 is moveable or deflectable from its first state 1000A (Fig. 7A) into its second state 1000B (Fig. 7B) upon insertion of the second mating member 200 into the first mating member 100, as shown, to allow passage of the second mating member 200 there-through and is moveable thereafter into its first state (Fig. 7A) to couple the second mating member 200 to the first mating member 100 of the releasably coupling assembly 400 as shown in Figs. 1A and 1D.

### Releasable Coupling System

As an overview of embodiments of the present invention, in some embodiments of the present invention a coupling system 500 is provided as shown in Figs. 1A and 1D, comprising a releasable coupling mechanism 300 for coupling two mating members 100, 200 In the embodiment shown, a releasable coupling system 500 is provided that comprises a first mating member 100, a coupling mechanism 300 comprising a coupling member 1000 associated with the first mating member 100 as shown in Fig. 1B, and a second mating member 200 that is receivable by the first mating member 100 to be secured thereto by the coupling member 1000.

For example, the releasable coupling system comprises a releasable coupling system 500 comprises a releasable coupling mechanism or releasable locking mechanism 300 that allows for releasably coupling two mating members 100, 200 that may comprise two devices 102, 202 such as two medical devices such as a sheath 120 and a dilator 220. Specifically, the releasable coupling system comprises a coupling mechanism comprising a coupling member 1000, a first mating member that houses or retains the coupling member 1000 (as shown in Fig. 1B), and a second mating member 200 that is receivable by the first mating member 100 (for example through an opening 112, to be coupled to the first mating member 100 by the coupling member 1000.

In one such embodiment, the first mating member 100 of the releasable coupling system 500 comprises a first handle portion 110 of a first medical device such as a sheath 120. In the example shown, the sheath 120 comprises a sheath hub 122 defining a housing 124, as shown in Fig. 1D, comprising an opening 112 (as additionally shown in Figs 1B and 1C) for receiving the second mating member 200 there-through as shown in Figs. 1A and 1D. Thus, in such embodiments of the releasable coupling system 500 of the present invention, the first mating member 100 comprises a housing 124, as illustrated in Fig. 1D. In some such examples, the coupling member 1000 is functionally coupled to the housing 124 of the first mating member 100, where it may not necessarily be attached or coupled but is functional to interact with the housing upon insertion and removal of the second mating member 200 from the first mating member 100, interaction with the housing is provided in additional detail herein and encompasses functional coupling as the term is used herein.

Additionally in the embodiment shown, the releasable coupling system 500 includes the coupling mechanism 300 comprising the coupling member 1000 ( such as a substantially oval disc shaped coupling member 1600 as additionally shown in Figs. 6A-6D) for securing the second mating member 200 that is inserted into the first mating member 100 as shown in Fig. 1D. In some examples, the coupling member 1000 is held within the first mating member 100. In some examples, the coupling member 1000 is seated or positioned within the mating member.

The releasable coupling system 500 additionally comprises the second mating member 200 that is configured to be inserted within and received by the first mating member 100. In some such embodiments, the second mating member comprises a second handle portion 210 of a second medical device 202 or a part of (such as a dilator 220, shown in Figs. 1C and 1D) therein, that is received by the opening 112. The second handle portion 210 comprises a dilator hub 222 comprises a second handle wider portion 226 for example such as ridge or a bump/ring 228 and a second handle groove portion or groove o224 (as shown in Figs. 2A(i), 2A(ii)) for receiving a portion of the coupling member 1000, such as snaps 1630 of a substantially oval disc shaped coupling member 1600 (as shown in Fig. 6A(iii)).

As noted herein above for the coupling mechanism 300, in the coupling system 500, the coupling member 1000 has a first state or configuration 1000A and a second state(or configuration 1000B as shown in Fig. 7A and 7B, where the coupling member 1000 is moveable from its first state 1000A (which for this particular example relates to a coupling member 1000 having a substantially oval disc shaped configuration 1600 where in the first state the coupling member has or retains its substantially oval disc shaped configuration as shown in Fig. 7A) where it is moveable into its second state 100B (where the substantially oval disc shaped coupling member 1600 moves or flexes into a substantially round or circular disc shaped configuration as shown in Fig. 7B) upon insertion of the second mating member 200 into the first mating member 100 to allow passage of the second mating member 200 there-through and is moveable thereafter into its first state (it goes back into its substantially oval disc shaped configuration as shown in Fig. 7A for this particular example) to couple the second mating member 200 to the first mating member 100. This mechanism is discussed further herein below.

As noted above, in some embodiments of the releasable coupling system, the second mating member 200 comprises a groove 226, and once the coupling member 1000 moves from its second state 1000B to its first state 1000A, the coupling member 1000 is receivable within the groove 226 of the second mating member 200 defining a co-operative engagement/arrangement 1226 there-between for releasably coupling the first 100 and second 200 mating members together.

In one specific example as discussed herein above with respect to Figs 7A- 7D, once the coupling member 1000 moves from its second state 1000B to its first state 1000A, specifically with respect to the substantially oval disc shaped coupling member 1600. I.e once the coupling member 1600 moves from its substantially round or circular disc shaped configuration in its second state as shown in Fig. 7B, back to its substantially oval disc shaped configuration 1000A as shown in Fig. 7A, it is receivable within the groove 226 of the second mating member 200 defining a co-operative engagement or arrangement 1226 there-between for releasably coupling the first 100 and second 200 mating members together.

In accordance with a releasable coupling system 500 of the present invention, in some embodiments the coupling member 1000 defines a first corresponding co-operative engagement feature or portion 1006 as shown in Fig. 6D(i), where in some examples, the first corresponding co-operative engagement feature 1006 comprises snaps 1030 of the coupling member 1000, such as snaps 1630 of the substantially oval disc shaped coupling member 1600. In some such examples, a second corresponding co-operative engagement feature/portion 206 is provided on the second mating member 200, which in some examples comprises a groove 226 of the second mating member 200, as shown in Fig. 6B and as shown in Figs. 2A(i), 2A(ii) as discussed previously herein.

In the releasably coupling system 500 as shown in Fig. 6B, in accordance with some examples of the present invention, wherein the first corresponding co-operative engagement feature or portion 1006 of the coupling member 1000 is operable to co-operatively engage to the second corresponding co-operative engagement feature or portion 206 of the second mating member 200 defining a co-operative engagement/arrangement there-between the first and second corresponding portions 1006, 206 to for releasably coupling the first and second mating members 100, 200.

In some such embodiments of the releasable coupling system 500, the releasable coupling mechanism 300 comprises and provides a translational locking mechanism for preventing the first and second mating members 100, 200 from moving translationally with respect to one another. In some such examples, the coupling member 1000 is functionally coupled to the housing 124 of the first mating member 100. In some such examples, the housing 124 for example in the case of a sheath 120 a housing base portion 124b and sheath hub cap 126. In some such examples the sheath hub 122 comprises a hub portion 125 that is coupled to the housing 124. The housing 124 for example as defined by housing base portion 124b and sheath hub cap 126 interacts with the coupling member 1000 to prevent translational movement of the second mating member 200 with respect to the first mating member 100.

As described herein above, as shown in Fig. 6A(iii) and 6A(iv), the coupling member 1000 of the coupling mechanism 300 interacts in co-operative engagement [at least functionally] with the second mating member 200, and functionally engages the two in the absence of force. The housing 124 interacts to prevent movement of the coupling member translationally in the proximal and distal directions [specifically the sheath hub cap 126 prevent proximal retraction of the second mating member 200 such as a dilator 220 and the base of the housing base portion 124b prevents distal movement of the mating member 200 such as the dilator 220 preventing further advancement of the dilator 220 into the sheath 120, and as such translationally locking the first mating member [such as the sheath 120] and the second mating member [such as the dilator 220].

### Releasable coupling member

As above, in embodiments of the present invention, a releasable coupling mechanism 300 is provided comprising a coupling member 1000, where the releasable coupling mechanism comprises a translational locking mechanism preventing the first and second mating members 100, 200 from moving translationally with respect to one another. In other words the coupling member 1000 enables translational locking of the first and second mating members 100, 200.

### General coupling member [General housing configuration - coupling member is functionally coupled to the housing]

As described herein above, some embodiments of the present invention provide a releasable coupling mechanism 300 where the coupling mechanism 300 further comprises a housing 124 of the first mating member 100 wherein the coupling member is functionally coupled to the housing 124 of the first mating member 100 and functions to retain the second mating member 200 once it is inserted into the housing 124. The coupling member 1000 is moveable to couple to the second mating member 200 to secure it to the first mating member. The coupling member 1000 is additionally configured to interact 124 with the housing to prevent removal of the second mating member 200 in the absence of force and to prevent translational movement of the coupling member 100 and thus the second mating member 200 coupled thereto, either distally prevent further advancement into the first mating member 100 or retraction distally. The interaction may comprise the coupling member 1000 abutting against the proximal inner surface of the housing 124 or a distal inner surface of the housing 124 to prevent translation thereof.

In some such examples of a releasable coupling mechanism 300 of the present invention, for example as shown in Figs. 1A-1D, the coupling member 1000 is held within the first mating member 100, for example within the housing 124.

### [Coupled/attached housing configuration- coupling member is coupled to the housing]

### Straight cantilever and u-shaped cantilever

In some such embodiments, where the coupling member 1000 is functionally coupled to the housing 124, the coupling member 1000 is coupled to a housing 124 of the first mating member 1000, with reference to Fig. 2A-2B, as well as Figs. 3A-3C, 4A-4C, Figs. A-5F. In some such examples, the coupling member 1000 is attached to the housing 124.

### Straight cantilever [coupling member is integrally formed with the housing]

With specific reference now to Figs. 2A(i)-2A(ii), and additionally 2B(i)-2B(ii), the coupling member 1000 is formed integrally with the housing 124 of the fist mating member 100, specifically a first 102 such as a medical device such as the sheath 120 as noted herein above. In some such embodiments of a releasable coupling mechanism 300, the coupling member 1000 comprises at least one cantilever 1010 as shown.

### Straight cantilever

In the example shown in Figs 2A(i)-2B(ii), the at least one cantilever comprises 1010 comprises at least one straight or simple cantilever 1200, as shown. In the specific instance of this example the at least one straight or simple cantilever 1200 is formed integrally with the housing 124 (for example with a cap thereof such as a sheath hub cap 126) shown in Fig. A(ii). In some such embodiments, the coupling member 1000 comprising the straight or simple cantilever 1200 comprises a flexible coupling member, it is able to flex from its first state or configuration 1000A [as shown in Fig. 2A(i)], for example radially outwards into its second state or configuration 1000B (as shown by directional arrows R), to allow the second mating member 200 to pass through the opening 112 of the first mating member 100, and then the straight or simple cantilever 1200 flexes back into its [Fig. 2A-2B]
In the illustrated example of the the at least one straight or simple cantilever comprises one or more retaining arms or members 1212 as shown in Fig. A(i). In some such embodiments, the one or more retaining arms or members 1212 terminate in one or more snaps 1230. In additional examples as shown in Figs. B(i),(ii) the at least one straight or simple cantilever 1200 comprises two or more straight or simple cantilevers 1200x, 1200y as shown (or at least two cantilevers 1200x, 1200y as shown).

In the embodiment shown, each of the two or more straight or simple cantilevers 1200x, 1200y is deflectable to move from the first state 1000A into a second state 1000B upon insertion of the second mating member 200 into the first mating member 100 to allow passage of the second mating member 200 there-through and the coupling member 1000 is capable of returning thereafter into the first state 1000A to couple the second mating member to the first mating member as shown in Fig. 2B(ii). As noted above, in some examples each of the two or more straight or (simple) cantilevers 1200 is deflectable in a radial direction to move between the first state 1000A and the second state 1000B.

Still furthermore, in the embodiments illustrated in Figs. 2A(i)-2B(ii) of the releasable coupling mechanism 300, each of the two or more straight or simple cantilevers 1200 is moveable in a plane P1 that is substantially in plane with the direction of advancement D of the secondary mating member 200 into the housing 124 for insertion therein. [Fig. 2A]. In other words each of the two or more straight or simple cantilevers 1200 is moveable in a plane P1 that is substantially in plane with a plane D1 that is substantially in plane with the direction of advancement D (or alternatively direction of removal) of the secondary mating member 200.

### U-shaped cantilevers [coupling member is attached to the housing]

In some embodiments, of the present invention, as discussed previously herein above, the coupling member 1000 is functionally coupled to the housing 124. In an example of this, the coupling member 1000 is held within the housing. In a specific instance, the coupling member 1000 is coupled to a housing 124 of the first mating member 1000, as discussed previously with reference to Fig. A(i)-2B(ii) above, as well as Figs. 3A-3C, 4A-4C, Figs. 5A-5F.

With reference now to Figs. 3A(i)-3C, 4A-4C, Figs. A-5F, similar to examples discussed previously herein above, the releasable coupling mechanism 300 of the coupling system 500 comprises a coupling member 1000 comprising at least one cantilever 1010 (as shown in Figs. 3B, 3C, and additionally Figs. 4B,4C and Figs 5B, 5C) to enable a second mating member 200 of the coupling system 500 to be coupled to the fist mating member 100, where the coupling member 100 is held within the housing 124. In some such examples, the coupling member 1000 is attached to the housing 124. In some such embodiments, the coupling member 1000 comprises at least one u-shaped cantilever 1300 as shown, which in some instances comprises an elastic u-shaped cantilever 1300 that is substantially elastic. In some such embodiments, as shown inFigs. 3A(i)-3C, 4A-4C, Figs. A-5F, the at least one u-shaped cantilever 1300 comprises a pair of u-shaped cantilevers 1300.

### Standard U-shaped cantilever

### coupling member coupled to the housing

With reference now to Figs. A(i) and 3A(iii), as well as Figs. 3D(i) and 3D(ii), the one or more one u-shaped cantilevers 1300 are held within the housing 124, specifically a housing base portion 124b that holds the substantially u-shaped cantilever 1300. In some examples, the housing base portion 124b is coupled to a hub portion 125. The substantially u-shaped cantilever 1300 is substantially held within and coupled to the housing base portion 124b. As shown, in some such embodiments, the substantially u-shaped cantilever 1300 is substantially exposed along a proximal face thereof and forms the proximal outer face of the first mating member 100 as shown in Fig. 3A(ii). In some such embodiments, the coupling member 1300 comprises a pair of standard u-shaped cantilevers 1301, as shown in Figs. A(i) to D(ii), where the distal to proximal cross-section view as shown in Figs. 3B and 3C defines substantially u-shaped cantilevers.

### Coupling member is contained within the housing

Alternatively, in some such embodiments of the present invention, as shown in Figs. 5A(i), 5A(ii), and 5A(iii), the pair of u-shaped cantilevers are substantially contained within the housing 124 defining substantially contained u-shaped cantilevers 1303, as shown in Fig. 5A(iv). Specifically, as shown in Figs. 5A(i)and 5A(iii) the u-shaped cantilevers 1300 are substantially contained within or retained by the housing 124 as defined by the housing base portion 124b and a hub cap 126 that together define the housing 124. In the illustrated embodiment, the housing base portion 124b is formed integrally with the hub cap 126, and thus the housing 124 of the first mating member 100 of the coupling assembly 400, is formed integrally and comprises a substantially unitary construction or piece. As such, the u-shaped cantilevers 1300 are substantially contained within the integrally formed housing 124.

Alternatively in some additional embodiments, as shown in Figs. D(i), 5D(ii), 5D(iii), and 5D(iv), the housing 124 is formed from a two part housing, comprising a housing base 124b and a separate hub cap such as a sheath hub cap 126 that are coupled together for example using a snap fit arrangement as shown in Fig.5E(i). As such, in some embodiments, the housing 124 comprises a housing base 124b and hub cap 126 and the u-shaped cantilevers are substantially contained within the housing base 124b and the hub cap 126 that together function to retain the substantially u-shaped cantilevers 1300 within the housing as shown in Fig. 5E(ii), defining contained u-shaped cantilever coupling members 1303 as above.

### Standard U-shaped cantilever - insertion and removal forces

In the example shown the U-shaped cantilevers have retaining snap arms or members 1312 that terminate in snaps 1330 as shown in Fig. 3B, 3C (as well as Figs 4B, 4C and 5B, 5). The snaps additionally have dual ramps 1333A, 1333B, where the first ramp 1333A of said dual ramps defines an insertion force and where the second ramp 1333B of said dual ramps defines the removal force where the ramp angles (Fi, Fr) create a component of force that compresses the snaps 1333. In some such examples, the angles on the first and second ramps (or in other words insertion and removal ramps) 1333A, 1333B may be varied to define respective insertion and removal forces. In some such examples, the first and second ramps 1333A, 1333B have varying (different) ramp angles defining varying (different) respective removal and insertion forces. In other examples, the first and second ramps 1333A, 1333b have substantially equivalent ramp angles defining substantially equivalent removal and insertion forces.

In the specific example shown in Figs. 3B, 3C, (and additionally in Figs. 5B, 5C and Figs. 5F(i), 5F(ii)) the insertion force and the removal force may be varied. In some instances, the force may be varied by a moment created on the snap arm 1312. Specifically as shown in Fig. 3B, 5B and 5F(i), as the second mating member 200 is advanced into the first mating member 100, the insertion force will create a moment on the snap arm or member 1312 which will increase the force required to overcome snap (in other words the force required to move the substantially u-shaped coupling member 1300 from its first configuration 1000A into its second configuration. In still other words, in some embodiments, the coupling member 1000 such as u-shaped coupling member 1300 is effectively biased in first state or configuration 1000A, and the moment on the snap arm 1312 will effectively increase the force required to overcome this bias in order to move the u-shaped coupling member into its second state or configuration.)

Conversely, upon removal of the second mating member 200 from the first mating member 100 as shown in Fig. 3C (and additionally Figs. C and 5F(ii)), the removal force will create a moment on the snap arm 1312, which will decrease the force required to overcome snap (in other words the force required to move the substantially u-shaped coupling member 1300 from its first configuration 1000A into its second configuration. In still other words, in some embodiments, the coupling member 1000 such as u-shaped coupling member 1300 is effectively biased in first state or configuration 1000A, and the moment on the snap arm 1312 will effectively decrease the force required to overcome this bias in order to move the u-shaped coupling member into its second state or configuration.) In some such embodiments, the angle of the second or removal ramp 1333B may be provided as a relatively steep angle for example in comparison to the angle of the first or insertion ramp 1333A, in order to increase the removal force to provide relatively uniform insertion and removal forces.

### Inverted U-shaped cantilever - coupling member contained with the housing

In other embodiments of the present invention, as illustrated in Figs. 4A(i), 4A(ii), 4A(iii) and 4A(iv), the coupling member 1000 comprising u-shaped cantilevers 1300 comprises a pair of inverted u-shaped cantilevers 1302 . As additionally illustrated in Figs. 4A(i), 4A(ii), 4A(iii) and 4A(iv), the substantially u-shaped cantilevers 1300 are held within the housing 124, specifically the base portion 125b of the housing 124, as shown and form the proximal face of the first mating member 100 (specifically with reference to Fig. 4A(iv). In these embodiments, the coupling members 1000 comprising the u-shaped cantilevers 1300 are coupled to the housing 124, specifically base portion 125b of the housing 124.

Similar to embodiments discussed previously with respect to Fig. 3B and 3C, in the specific example shown in Figs. 4B, 4C, the insertion force and the removal force may be varied. In some instances the force may be varied as a result of a moment created on the snap arm 1312. Specifically as shown in Fig. 4B, as the second mating member 200 is advanced into the first mating member 100, the insertion force will create a moment on the snap arm or member 1312 which will decrease the force required to overcome snap (in other words the force required to move the substantially u-shaped coupling member 1300 from its first configuration 1000A into its second configuration. In still other words, in some embodiments, the coupling member 1000 such as u-shaped coupling member 1300 is effectively biased in first state or configuration 1000A, and the moment on the snap arm 1312 will effectively decrease the force required to overcome this bias in order to move the u-shaped coupling member into its second state or configuration.)

Conversely, upon removal of the second mating member 200 from the first mating member 100 as shown in Fig. 4B, the removal force will create a moment on the snap arm 1312, which will increase the force required to overcome snap (in other words the force required to move the substantially u-shaped coupling member 1300 from its first configuration 1000A into its second configuration. In still other words, in some embodiments, the coupling member 1000 such as u-shaped coupling member 1300 is effectively biased in first state or configuration 1000A, and the moment on the snap arm 1312 will effectively increase the force required to overcome this bias in order to move the u-shaped coupling member into its second state or configuration.)

In some such embodiments, the angle of the first or insertion ramp 1333B may be provided as a relatively steep angle for example in comparison to the angle of the second or removal ramp 1333A, in order to increase the required insertion force to provide relatively uniform insertion and removal forces.

### U-shaped cantilever - structure

In some such embodiments of the present invention, as shown in Figs. 3A(i)-3D(ii), 4A(i)-4C, 5A(i)-5F(ii)], the coupling member 1000 comprising the u-shaped cantilever 1300 comprises a moveable coupling member 1000 where it is moveable between the first and the second states 1000A, 1000Bf. Additionally, in the examples shown, the coupling member 1000 comprises a flexible coupling member, specifically the u-shaped cantilever 1300 is flexible enabling the cantilever arms 1312 to flex between the first state 1000A and the second state 1000B. In still further examples of the coupling member 1000 that embodies the u-shaped cantilever 1300, the u-shaped cantilever 1300 and thus the cantilever arms 1312 comprise a resilient material, thus defining a resilient coupling member 1000.

In still further embodiments of the present invention, the coupling member 1000 comprises an elastic coupling member that is elastically deformable to transition between the first and the second states 1000A, 1000B and substantially avoids plastic deformation. In some such examples, the coupling member 1000 remains in the elastic region of the strain curve. Thus, in some embodiments where the u-shaped cantilever 1300 is provided as outlined herein, the cantilever sections, specifically the cantilever arms 1312 are elastically deformable.

In some such embodiments, each of the pair of u-shaped cantilevers 1300 is deformation, specifically elastically deformable to move from the first state 1000A into a second state 1000b upon insertion of the second mating member 200 into the first mating member 100 to allow passage of the second mating member 200 there-through and the coupling member 1000 defined by the u-shaped cantilever 1300 is capable of returning thereafter into the first state 1000A to couple the second mating member 200 to the first mating member 100. In some such examples, each of the pair of u-shaped cantilevers 1300 is elastically deformable in a radial direction to move between the first state 1000A and the second state 1000B, for example as shown in Figs. 3B-3C, 4-4C, 5B-5C).

In some such embodiments, each of the pair of u-shaped cantilevers 1300 is moveable in a plane P1 as shown in Fig. 3B (but is applicable to Figs. 3C, 4B,4C, 5B, 5 as well) that is substantially in plane with the direction D of advancement of secondary mating member into the housing for insertion therein (or removal therefrom).

In other words each of the pair of u-shaped cantilevers 1300 is moveable in a plane P1 that is substantially in plane with a plane D1 that is substantially in plane with the direction of advancement D (or alternatively direction of removal) of the secondary mating member 200 into/from the first mating member 100.

Alternatively in some embodiments, the pair of u-shaped cantilevers 1300 is moveable in a plane p2 that is perpendicular to the direction D of advancement of second mating member200 into the housing 124 for insertion therein (extending into and out of Fig.3B as shown.

### Substantially annular cantilever - coupling member contained with the housing

In alternate embodiments of the present invention, as shown in [Figs. 6A(i)-6F(iv), 7A-7D, 8A-8F(iii), 13A-13D, 14A-14F(iii)], a releasable coupling member 1000 is provided where the coupling member 1000 is substantially contained or retained within the housing 124. In the embodiments shown the coupling member 1000 is substantially free floating or loose within the housing 124.

Specifically with reference to Figs. 6A(i)-6F(iv), in accordance with an embodiment of the present invention a coupling mechanism 300 is provided comprising a coupling member 1000 comprises at least one cantilever 1010 (or in other words a cantilever portion or segment 1010), as shown in Fig. 6D(i). In the illustrated example, the at least one cantilever 1010 comprises a simply supported beam configuration. Specifically as shown, the at least one cantilever 1010 comprises two cantilevers 1010 (or in other words comprises a pair of cantilever portions or segments).

With reference again to Fig. 6D(i), a pair of cantilevers 1010 that are coupled together. As shown in some embodiments, the two cantilevers 1010 comprise two substantially straight segments (S) (or in other words substantially straight portions or sections) that are coupled together using one or more arcuate segments (C), as shown in Fig. 8A. In a specific instance of this as shown, each of the two straight segments (S) of the cantilevers 1010 are coupled together at each of their respective ends by an arcuate (C) segment.

In some of the embodiments shown, each of the two cantilevers 1010 comprise deflectable portions or regions, where each of these deflectable portions are defined by a simply supported beam configuration. In the specific configuration shown, the substantially straight segments (S) define the deflectable portions where maximum deflection (M) is substantially along the mid-point of the substantially straight segments (S). In some such examples, each of the deflectable portions comprise one or more retaining arms 1612 where the one or more retaining arms comprise one or more snaps 1630.

In one specific example, the coupling member 1000 in accordance with a coupling mechanism 300 of the present invention, comprises a substantially annular disc shaped configuration 1601, [or in other words the coupling member substantially comprises an annular disc] similar to the configurations shown in Figs. 7A and 7B. In a specific instance of this example, the coupling member 1000 comprises a substantially oval disc shaped configuration [or in other words the coupling member substantially comprises an oval disc], with reference again to Figs. 6D(i) and Fig. 8A. As a feature of this, the substantially oval disc shaped coupling member 1600 comprises one or more retaining arms 1612 that terminate in one or more snaps 1630.

In the embodiments illustrated in Figs. 6A(i)-6F(iv), 7A-7D, 8A-8F(iii), 13A-13D, 14A-14F(iii), the substantially oval disc shaped coupling member 1600 is functionally coupled to the housing 124 but in this particular embodiment it is substantially not coupled or remains unattached or unengaged to the housing during use so that it is moveable feely within the housing 124. Specifically with reference to Figs. 6D(ii), 6E(i) and 6E(ii), the the substantially oval disc shaped coupling member 1600 is housed within a housing base portion 124b of a sheath hub 122 and is retained therein by the sheath hub cap 126. As such, the combination of the housing base portion 124b and the sheath hub cap 126 form an enclosure to enclose the substantially oval disc shaped coupling member 1600 therein as additionally shown in Fig. 6F(i).

As outlined previously herein above with reference now to Fig. 6E(ii), (as well as Fig. F(i) 6F(ii), 6F(iii) which show an alternate second mating member 200) the housing 124, for example as defined by housing base portion 124b and sheath hub cap 126 interacts with the substantially oval disc shaped coupling member 1600 to prevent translational movement of the second mating member 200 with respect to the first mating member 100, once the second mating member 200 is inserted into the first mating member 100 as shown in Fig. F(iv).

In some such examples , The housing 124 interacts to prevent movement of the coupling member translationally in the proximal and distal directions [specifically the sheath hub cap 126 prevent proximal retraction of the second mating member 200 such as a dilator 220 and the base of the housing base portion 124b prevents distal movement of the mating member 200 such as the dilator 220 preventing further advancement of the dilator 220 into the sheath 120, and as such translationally locking the first mating member [such as the sheath 120] and the second mating member [such as the dilator 220]. Furthermore, as shown in Figs. 6B and 6C, the substantially oval disc shaped coupling member 1600 and specifically the snaps 1630 thereof will rub against the distal face 124f of the housing base portion 124b, and the friction will thereby increase the insertion force as shown in Fig. 6B. Conversely, the substantially oval disc shaped coupling member 1600 and specifically the snaps 1630 thereof will rub against the proximal face 126f of the sheath hub cap 126, and the friction will thereby increase the removal force. In some such examples, the substantially oval disc shaped coupling member 1600 is functionally coupled to the housing 124 in that it may not necessarily be attached or coupled (as provided in this embodiment) but is functional to interact with the housing upon removal or insertion of the second mating member 200 to facilitate insertion and removal of the second mating member 200 and coupling it to the first mating member 100.

In some such embodiments of the present invention, the coupling member 1000 such as the substantially oval disc shaped coupling member 1600 comprises a substantially flexible coupling member 1000. In specific embodiments of the present invention, as shown in Figs. 7A-7D, the substantially oval disc shaped coupling member 1600 is deformable, wherein the coupling member comprises an elastic coupling member that is elastically deformable.

In other words in some embodiments of the present invention, the coupling member 1000 substantially comprises a snap ring or band 1601, which may have a configuration as shown in Figs. 7A or 7B. In a specific example, the coupling member 1000 comprises a substantially oval snap ring or band 1600 as additionally shown in Fig. 8A. In some such embodiments, the coupling member 1000 comprises a substantially annular cantilever.

### Substantially annular cantilever - function

In the embodiments shown where the coupling member 1000 comprises a substantially oval disc shaped coupling member 1600, the coupling member 1000 that has a substantially oval disc-shaped configuration in its first state 1000A (Fig. 7A) and substantially round or circular disc-shaped configuration in its second state 1000B (Fig. 7B). The substantially oval disc shaped coupling member 1600, is deflectable to move (for example radially) from its oval disc-shaped configuration in its first state or configuration 1000A into its substantially round or circular disc-shaped configuration 100B in its second state upon insertion of the second mating member 200 into the first mating member 100, as shown in Fig. 6B and Fig. 8B, to allow passage of the second mating member 200 there-through and the a substantially oval disc shaped coupling member 1600, is capable of returning thereafter into the first state 1000A defined by the oval configuration to couple the second mating member 200 to the first mating member 100, as shown in Fig. 6C and Fig. 8F(iii).

Alternatively, the substantially oval disc shaped coupling member 1600 (or alternatively substantially oval snap ring or band 1600) has a first engaging configuration in its first state 1000A which comprises a substantially oval disc shaped configuration [or a substantially oval configuration] and wherein the coupling member has a second non-engaging configuration in its second state 1000B which comprises a substantially circular disc shaped configuration [or a substantially round or circular configuration]. The substantially oval disc shaped coupling member 1600 is moveable (for example radially) into its second non-engaging configuration comprising the substantially circular disc shaped configuration upon insertion of the second mating member 200 into the first mating member 100 (Figs. 6B and 8(i)) to enable the second mating member 200 to advance therein and is moveable thereafter into its first engaging configuration comprising the substantially oval disc shaped configuration (as shown in Fig. 6C, Fig. F(iii)) to couple the second mating member 200 to the primary or first mating member 100.

In some such examples, with reference to Figs. 6F(i), 6F9ii), 6F(iii), 6F(iv)the substantially oval disc shaped coupling member 1600 (or alternatively substantially oval snap ring or band 1600) is elastically deformable to move between the first state 1000A and second state 1000B. More specifically in some examples, the substantially oval disc shaped coupling member 1600 (or alternatively oval snap ring or band is moveable outwards, for example radially, into its second non-engaging configuration having a substantially round configuration 1000B upon insertion of the second mating member 200 into the housing of the first mating member 100 [to allow the second mating member 200, specifically to allow a raised portion of the dilator hub 222 (such as a bump or a ring 228) to advance into the housing 124 of the first mating member 100 (to pass past the substantially oval disc shaped coupling member 1600 (or alternatively resilient snap ring or band) specifically past one or more snaps 1630 positioned along the body of the coupling member]. The substantially oval disc shaped coupling member 1600 is moveable thereafter into its first engaging configuration comprising a substantially oval configuration 1000A to couple the second mating member 200 to the primary or first mating member 100 (in other words the circular resilient ring or band is then configured to change or move back into its original substantially oval shape or configuration (a substantially oval snap ring or band) and thus its original position (allowing it to be positioned within a groove 226 of the dilator hub 222.

As noted herein, wherein the substantially oval disc shaped coupling member 1600 is elastically deformable in a radial direction [R] (as outlined in Figs. 6B, 6C and Figs 8B, 8C) to move between the first state 1000A and the second state1000B.

### Snap Force being independent of the hub length

In some embodiments of the each of the pair of cantilevers 1010 of the substantially oval disc shaped coupling member 1600 (as shown in Fig. D(i), Fig. 8A) is moveable in a plane [P2] that is perpendicular to the direction of advancement [D] of secondary mating member 200 into the housing 124 for insertion therein.

In some such embodiments, the snap force [i.e. the force required for coupling the second mating member 200 to the first mating member 100 using the coupling mechanism 300] is independent of a proximal length of the first mating member 100. In other words, the force required to enable the substantially oval disc shaped coupling member 1600 to move from its first state 1000A to its second state 1000B to enable coupling, is independent from the length of the first mating member 100, such as a length of a medical device hub such as a dilator hub 122. As a result in some such examples, the frictional forces from insertion of the second mating member into the first mating member 100 against the coupling member 1000 can be minimized and as a result there is reduced drag. As a result additional frictional forces are substantially not introduced and are not additive to the snap force required to deflect the coupling member 1000 to enable coupling or in other words snapping in and snapping out of the second mating member 200. As such the insertion and removal forces are substantially determined by the coupling member 1000 (specifically the substantially oval disc shaped coupling member 1600 with reference to Figs 8A, Fig.8F(ii)) which may alternatively be referred to as a racetrack.

In some embodiments of the present invention the insertion and removal force [Fi] and [Fr] are about 15 Newtons. In other embodiment.

### Coupling member is flipped sideways so it isn't obstructing view of the valve

In some embodiments of the present invention, as shown in Fig. F(i) and Fig. 8Ff(iii), the first mating member 100, specifically comprising a first device 102 comprising a medical device (such as a sheath 120) has a medical device hub 104 that further comprises a valve 150, wherein the coupling member 1000 (specifically the substantially oval disc shaped coupling member 1600) is oriented in a plane p2 that is perpendicular to the direction of advancement D of the second mating member 200 (as shown in Fig. 8A), whereby it does not substantially hinder visibility of the valve 150 from the user. In other words the coupling member 1000 in the orientation shown does not substantially require use of the hub length (L) for example as defined by the length of the housing 124 as shown in Fig. F(iii), for the cantilever 1010 lengths. In some such embodiments, the coupling mechanism 300 utilizes the width of the housing 124, as defined by the real estate provided by the diameter of the housing 124. This ensures that the snapping force is independent of the hub length (L) and helps optimize visibility of the valve 150 through the opening 112 for the user by allowing it be positioned in relative proximity to the opening 112.

### Insertion Force and Removal Force being substantially equivalent/uniform

In some embodiments of the present invention, as also discussed previously hereinabove, with reference now to Figs. 8F(i), 8F(iii), The coupling member 1000, specifically the substantially disc shaped coupling member 1600 as noted previously comprises cantilever arms or retaining arms or members 1612 that have snaps 1630 for example along the mid portion of the retaining arms or members 1612 at the region of maximum deflection. In some such examples of releasable coupling mechanism 300 the snaps 1630 additionally have dual ramps 1633A, 1633B, where the first ramp 1333A of said dual ramps defines an insertion force and where the second ramp 1333B of said dual ramps defines the removal force where each of the ramp angles (Fi, Fr) create a component of force that compresses the snaps 1630.

In some embodiments, the insertion force [Fi] for inserting the second mating member 200 into the coupling mechanism 300 to be coupled to the first mating member 100 by the coupling member 1000 (specifically the substantially oval disc shaped coupling member 1600) is substantially equivalent to the removal force [Fr] for removing the second mating member from coupling mechanism to be disengaged from the first mating member. [Fig. -8F] In some such examples, the angles on the first and second ramps (or in other words insertion and removal ramps) 1633A, 1633B may be varied to define respective insertion and removal forces. In some such examples, the first and second ramps 1633A, 1633B have varying (different) ramp angles defining varying (different) respective removal and insertion forces, as shown in Fig. 8C. For example Fig. 8C provides a greater or steeper angle on the first ramp or insertion ramp 1633A compared to the ramp angle for the second or removal ramp 1633B, which would increase the insertion force [Fi] in comparison to the removal force (for example where the insertion force is the force required to utilize the coupling mechanism for example to snap the second mating member 200 in using the snap mechanism 300 as shown). As such, in the specific example shown in Figs. 8C, embodiments of the present invention provide a means to vary or in other words provide a varying or different insertion force [Fi] and the removal force [Fr].

### Tuning of the insertion force and removal force

As such some embodiments of the present invention, the insertion force [Fi] (for inserting the second mating member 200 into the coupling mechanism 300 to be coupled to the first mating member 100 by the coupling member 1000, specifically substantially oval disc shaped coupling member 1600) and the removal force [Fr] (for removing the second mating member 200 from coupling mechanism to be disengaged from the first mating member 100) are tunable as shown in Figs. 8A-8C, as well as Fig. D(i). As such, some embodiments of the present invention, provide a mechanism to tune or control the insertion and removal forces (Fi, Fr) for example by changing the respective ramp angles for insertion and removal ramps 1633A, 1633B. In some such examples, the coupling member 1000 such as a substantially disc shaped coupling member 1600, one or more orientation keys 1480 are provided so the substantially disc shaped coupling member 1600 can be inserted in the desired orientation with the appropriate insertion ramp 1633A and 1633B being oriented to have the appropriate insertion and removal ramp angles as desired as shown in Fig. 8E(i), 8E(ii) and 8E(iii). This may facilitate assembly where the removal ramp angle may be different than the insertion ramp angle.

### Providing substantially equivalent insertion force and removal force

In other words the first and second ramps 1633A, 1633B may be varied to define respective insertion and removal forces (Fi, Fr).In other examples, the first and second ramps 1633A, 1633B have substantially equivalent ramp angles defining substantially equivalent removal [Fr] and insertion forces [Fi] respectively, as shown in Fig. 8B.

### Snap force may be varied

In some embodiments of the present invention, as shown in Fig. 8D(ii), 8E(ii) and 8E(iii), the snap force (for example the force required to snap the second mating member 200 to the first mating member 100 using the coupling member 1000, specifically the substantially oval disc shaped coupling member 1600) for example as defined by the insertion force [Fi] and/or the removal force [Fr] can be dialed in (or changed) by varying the wall thickness [T]and the snap height. In some such examples, the snap overlap [X] is defined as the overlap between the snap 1630 and the portion of the second mating member 200 that is inserted into the first mating member 100, for example for a dilator 220, the portion of dilator 220 defining the groove 226 of the dilator 220 and/or the ridge or bump 228 of the dilator hub 222, with reference to Fig. F(ii). In one such example, the wall thickness T is about 0.75mm and the snap overlap X is about 0.5mm. In another example, the wall thickness T is about 1.0mm and the snap overlap is about 1.0mm.

### Coupling member remains within the elastic region

In some such embodiments of the present invention as shown in Figs. 7A, 7B and additionally shown in Figs. 7C and 7D, the substantially disc shaped coupling member 1600 of the releasable coupling mechanism 300 substantially remains in the elastic region upon deformation, for example as it flexes between the first state 1000A and the second state 1000B. In some such embodiments of the present invention, with reference again to Figs. 7A-7D as well as Figs. 8B-8C, wherein the insertion force [Fi] for inserting the second mating member 200 into the coupling mechanism 300 to be coupled to the first mating member 100, and the removal force [Fr] for removing the second mating member 200 from coupling mechanism 300 to be disengaged from the first mating member, are substantially uniform over multiple uses of the coupling mechanism 300. In other words, the insertion force [Fi] and the removal force [Fr] remain substantially unchanged over multiple uses. In some embodiments, the ability of coupling member 1000 (for example the substantially oval disc shaped coupling member 1600) to remain in the elastic region during deformation and flexion substantially facilitates that the insertion and removal forces (Fi, Fr) remain substantially constant or unchanged over multiple uses.

In some such examples, the substantially oval disc shaped coupling member 1600 (racetrack component or snap component) is able to flex since it is free floating within the housing 124, and the sheath hub length L is minimized since the flexing is in a radial direction. The racetrack is formed integrally or in other words has continuous geometry which facilitates reduction in stress concentrations. Addtionally the snap force can be dialed in or changed or tuned with altering wall thickness T and snap overlap or snap engagement X. In some such examples, the substantially oval disc shaped coupling member 1600 comprises a polycarbonate. In additional examples, the substantially oval disc shaped coupling member 1600 may comprise a resilient material. In another example the material is ABS Acrylonitrile butadiene. In some embodiments the strain seen by the coupling member 1000 such as the substantially oval disc shaped coupling member 1600 during flexion between first and second states 100A, 100B is less than about 6%, where the coupling member 1000 would require a strain of greater than about 6% before plastic deformation occurs. Above this strain value the coupling member 1000 would be plastically deformable but the structure of the coupling member 1000 prevents this by providing elastic deformation over multiple uses. In some such example, the strain the coupling member 1000 sees is substantially dependent on the structure (for example it is based on the structure of the substantially oval disc shaped coupling member 1600 which has a substantially racetrack structure based on the geometry) which allows for a less than 6% strain value.

In some embodiments as shown in Figs. 13A, 13B, 13E the substantially oval disc shaped coupling member is oriented so that the cantilever arms 1612 are oriented so that they are parallel to the flat faces 408 of the hub cap and the snaps 1630 are positioned along the flat faces. In some embodiments as shown in Figs. 13C and 13D, 13F the substantially oval disc shaped coupling member is oriented so that the cantilever arms 1612 are oriented so that they are perpendicular to the flat faces 408 of the hub cap, so the snaps 1630 are positioned adjacent the guides 402.

In an alternative embodiment of the present invention, a coupling member 1000, specifically substantially disc shaped coupling member 1600 is provided in a first mating member 100 that comprises a steerable sheath 190 as shown in Figs. 14A, 14B, 14C,14D, 14E and 14F, where the substantially disc shaped coupling member 1600 is contained or retained within the housing 124 (formed by a housing base 124b and a hub cap 126) and may comprise additional component to contain the coupling member in the housing. The coupling member is substantially free floating in the housing 124, where the coupling member 1000 (substantially disc shaped coupling member 1600) is configured to couple the second mating member comprising the dilator 220 to the steerable sheath 190.

### General Audible feedback

In some embodiments of the present invention, the coupling member 1000, sich as coupling members (straight cantilever coupling member 1200), (u-shaped coupling member 1300) and (substantially oval disc shaped coupling member 1600), configured to generate an audible feedback upon movement between the first state 1000A and second state 1000B, with reference to Figs. 2A(ii), 2B(ii), 3B,3C, 4B. 4C, 5B,5C In some such examples the coupling member 1000 (specifically each of the straight cantilever coupling member 1200, u-shaped coupling member 13000 and substantially oval disc shaped coupling member 1600), interact with the second mating member 200 upon insertion into the first mating member100 to generate audible feedback. In some such example, the respective snaps 1230, 1330 and 16300 of the respective coupling members interact with a portion of the second mating member 200 (such as groove 226) upon once the coupling member returns from its second state 1000B to the first state 1000A generating an audible click indicating insertion and coupling of the second mating member 200 to the first mating member 100. Similarly in some such examples, the respective snaps 1230, 1330 and 16300 of the respective coupling members interact with a portion of the second mating member 200 (such as ridge or bump 228)upon once the coupling member returns from its second state 1000B to the first state 1000A upon removal of the second mating member 200 from the first mating member 100 to generate an audible click indicating removal and decoupling of the second mating member 200 from the first mating member 100.

### Alternate claim for the racetrack embodiment

In some embodiments of the present invention, specifically with reference to Figs. 6A(i)-6F(iv), 7A-7D, 8A-8F(iii), a releasable coupling system 500 is provided comprising a first mating member 100 defining an opening 112 for receiving a second mating member 200 there-through. In some such examples the second mating member 200 has a first diameter (for example of the portion of the dilator 220 defining the groove 226) defining an effective diameter that is smaller than the opening 112 enabling it to be advanced into the first mating member 100 through the opening 112. The system 500 further comprises a means for increasing the effective diameter of the second mating member 200, the means comprising a coupling member 1000 (specifically the substantially oval disc shaped coupling member 1600) positioned/located within the first mating member 100. Upon insertion of the second mating member 200 into the first mating member 100 through the opening 112 thereof, the coupling member 1000 (specifically the substantially oval disc shaped coupling member 1600) is coupled to the second mating member 200, thereby modifying [increasing] the effective diameter of the second mating member 200 (for example at the groove 226) such that the effective diameter of the second mating member 200 is larger than the opening 112 of the first mating device 100 preventing it from exiting therefrom/moving translationally therein. Additionally the effective diameter of the second mating member 200 is larger than a distal opening in the housing 124 of the first mating member 100. Thus, the coupling member 1000 when functionally coupled to the second mating member prevents or blocks the movement of dilator hub 122 out of the sheath hub 122 in the absence of force, thereby preventing the dilator hub from exiting by interacting with both the distal face and the proximal inner faces of the housing 124.

### Alternatives

In other words, the embodiments shown in Figs. 1A-1D, specifically with reference to Fig. 1B, the flexible coupling mechanism 300 additionally a flexible locking member or component 1000 that is seated within the first handle housing such as the sheath housing 124 for releasably coupling the second handle portion 210 to the first handle portion 110. The flexible locking member or component has a first configuration 1000A which can also be the locking configuration as shown in Fig. 7A, and Fig. 6C and a second non-locking configuration 1000B (Fig 7B). Upon initial advancement of the second handle portion 210 into the first handle portion, the flexible locking member or component 1000 is moveable outwards from its first locking configuration 1000A into its second non-locking configuration 1000B out of the path of the second handle wider portion such as ridge or bump 228 upon interaction therewith, and upon further advancement of the second handle portion 210 into the first handle portion 110, the flexible locking member or component 1000 is moveable inwards into the second handle groove portion 226 from its second non-locking configuration into its first locking configuration as shown in Fig. 7A, in order to releasably couple the dilator hub 222 to the sheath hub 122, whereby the flexible locking member or component 1000 is moveable into its second non-locking configuration upon exertion of force as the dilator hub 222 is pulled to enable disengagement of the releasable locking mechanism. In some examples the flexible locking member or component has snaps 132.

### Rotational Locking Mechanism

In accordance with some embodiments of the present invention a coupling mechanism is provided that allows two devices to be coupled together so they can be maneuvered or manipulated together thereby forming a rotational locking assembly or system that comprises the two devices. In some such examples, the coupling mechanism comprises a locking mechanism to allow one or more of rotational and/or translational locking of the two devices. In addition to coupling mechanism 300 that function as translational locking mechanisms as described herein above, some embodiments of the present invention additionally provide for rotational locking mechanism 400 in addition to the releasable coupling mechanism 300. Some emboidments may include one or more of the releasable coupling mechanism and rotational locking mechanism.

In some such examples, with reference now to Figs. 9A(i), 9A(ii), 9A(iii) and 9A(iv), the coupling mechanism comprises a rotational locking mechanism 400 comprising guides and/or corresponding co-operating features (such as guides 402, 404 and 406 within or along the two devices for enabling coupling of a sheath 100 and a dilator 200) to allow the two devices to be maneuvered or manipulated together. The present invention overcomes problems with prior art systems in terms of meeting the need in the art for: a) allowing the user to have the ability to rotate the connected devices using either the sheath hub or dilator hub or handle, and additionally b) to allow the dilator to be oriented in the same direction as the curve of the sheath (for example in embodiments where the dilator is stiff).

In one embodiment of the present invention, a rotational key mechanism or rotational locking mechanism 400 has been added to the first and second mating members 100, 200 dilator and sheath hubs 104, 204, as discussed above and as shown in Figs. 1A and 1C, allowing them to be maneuvered together such as rotated or torqued together. The coupling mechanism comprising a rotational locking mechanism 400 as shown, ensures proper alignment (for example between the first and second mating members 100,200 such as the sheath and dilator hubs 122, 222 and thus the dilator and sheath 120, 220) as shown in Fig. 9A(i) and 9A(iv) and thus provides the assembly of the sheath 120 and dilator 220 to be maneuvered together and hence provides the assembly with the ability to transmit torque.

In the particular example shown in Figs. 1Aand 1C, the sheath hub 122 comprises guides 402 which are sides that funnel in towards the valve In one such embodiment the housing 124 of the first mating member 100, specifically the sheath 120 comprises one or more guides 402 comprising angles or tapered guides or surfaces 406 as shown in Fig. 9A(iii). The first mating member additionally comprises one or more keying surfaces or features 404 such as adjacent flat surfaces 408 comprising keying and torque transfer surfaces. In some such embodiments keying and torque transfer is provided on or using the flat faces or surfaces 408. In some such examples, one or more of the guides 402 and the keying features 404 define a first corresponding engagement feature 460 of the locking mechanism 400. The locking mechanism further comprises corresponding guide portions 202 on the dilator hub 222 that are angled that interact with the guides 402 to guide the dilator 222 into the sheath hub 122. Additionally the dilator hub 222 comprises one or more corresponding keying surfaces 204 that correspond to the keying surfaces or features 404 of the sheath. In some such embodiments, one or more of the keying surfaces 204 and guide portions 202 of the dilator 220 provide a second corresponding engagement feature 260 for the rotational locking mechanism 400 to enable co-operative coupling for example to enable rotational locking. The sheath hub further comprises flat faces 404 to provide keying and torque transfer. In some such embodiments the user can pivot the dilator hub 220 for example 3-9 O-clock to loosen the snap. In some such embodiments the force to disengage the rotational locking mechanism 400 is greater than about 10Newtons. In some embodiments it is about 15 Newtons.

In some such embodiments, due to the angled face 209 on the front of the dilator, when the dilator is torqued the corner edges touch. In some examples, the corresponding flat faces may not mate when torqued. The torque being applied on the angle generates an unsnapping force for the rotational locking mechanims. When the torque is sufficient to generate enough unsnapping force, then the rotational lock disengages and the dilator 220 unsnaps from the sheath 120.

In an additional embodiment of the present invention as shown in Figs. 9B, and 9C, a rotational locking mechanism is provided a first corresponding engagement feature on the first mating member 100 comprising a key 428 for example formed in a portion of the housing 124 such as the hub cap 126) and a second corresponding engagement feature comprising a rib 228 on the second mating member 200.

In an additional embodiment of the present invention as shown in Figs. 10A, 10B and 10C, a rotational locking mechanism is provided a first corresponding engagement feature on the first mating member 100 comprising a key 428 (for example formed in portion of the housing 124 such as the cover or hub cap 126) and a second corresponding engagement feature comprising a rib 228 on the second mating member 200.Additionally chamfers are provided on the key 428 and radii or curved edges on the rib 228 to facilitate guiding the second mating member 200 the dilator 220 into the first mating member 100, i.e. the sheath 120. In some such examples, the key 428 comprises a slot in the cover or hub cap 126 and the slot in the hub cap 126 and the rib 228 have corresponding matching angles which may facilitate surface contact.

In an additional embodiment of the present invention as shown in Figs. 11A, and 11B, and 12A and 12B a rotational locking mechanism is provided a first corresponding engagement feature on the first mating member 100 comprising a key 428 (for example formed in a portion of the housing 124 such as the hub cap 126) [which in Figs. 11A, 11B comprises a slot in the hub cap 126, and in Figs 12A, 12B comprises at least two grooves]. The rotational locking mechanism 400 further comprises a second corresponding engagement feature comprising a rib 228 which may be angled on the second mating member 200. In Figs 12A, 12B, two rib sections 228 are provided that correspond to the two grooves 428.

In some such embodiments a rotational locking mechanism is provided comprising, a first corresponding engagement feature on a first mating member, a second corresponding engagement feature on a second mating member, wherein the first and second corresponding features enable rotational locking. In some such examples, the rotational the first corresponding feature comprises one or more guides. In one such example, the one or more guides comprise a funnel. In some such embodiments, the first corresponding feature comprises a keying feature. In some such embodiments the keying feature comprises a substantially flat face. In some such embodiments, the second corresponding feature comprises one or more guide portions that are configured to mate with the one or more guides of the first corresponding feature of the sheath. In some such embodiments, the second corresponding feature comprises keying surfaces that correspond to the keying features of the first corresponding feature.

Figs. 2-111 additionally show additional embodiments of coupling mechanisms in accordance with various embodiments of the present invention.

### Various embodiments of coupling members

In some embodiments of the present invention, the coupling member comprises a flexible coupling member. In some embodiments, the coupling member comprises a resilient coupling member. In still some examples, the coupling member comprises an elastic coupling member that is elastically deformable.

### Cantilever coupling member

In some embodiments, the coupling member comprises at least one cantilever.

### Straight cantilever

In some embodiments, the coupling member comprises at least one straight or simple cantilever. In some such examples, the at least one straight or simple cantilever is coupled to [a housing of the first mating member. In some embodiments, the coupling member comprises two or more straight or (simple) cantilevers.

### U-shaped cantilever

In some embodiments, the coupling member comprises at least one u-shaped cantilever. In some such embodiments, the at least one u-shaped cantilever comprises at an elastic u-shaped cantilever. In one example, the coupling member comprises a pair u-shaped cantilevers. In a specific instance, the coupling member comprises a pair of inverted u-shaped cantilevers.

### Substantially disc-shaped cantilever

In another embodiment, the coupling member comprises a substantially disc shaped cantilever or a substantially annular cantilever or substantially annular coupling member. In one example the coupling member comprises a substantially disc shaped configuration. In one example the coupling member comprises a substantially oval disc shaped configuration. In one embodiment, the coupling member substantially comprises a snap ring or band (or substantially annular snap ring or band). In a specific example of this, the coupling member comprises a substantially oval snap ring or band.

### General Audible feedback

In some embodiments, the coupling member is configured to generate an audible feedback upon movement between its first state and second state. In some such embodiments, the coupling member interacts with the second mating member upon insertion into the first mating member to generate audible feedback. In some embodiments, the coupling member interacts with the second mating member upon once the coupling member returns from its second state to the first state generating an audible click.

### Alternatives

In some embodiments, a locking mechanism is provided comprising a locking (member or component) held (seated/positioned) within a locking device for releasably coupling a second device thereto, where the second device is receivable by the locking device, wherein the locking (member or component) has a first state (or configuration) and a second state (or configuration), and wherein the locking member is moveable from its first state into its second state upon insertion of the second device into the locking device to allow passage of the second device there-through and is moveable thereafter into its first state to couple the second device to the locking device.

In some embodiments, a releasable locking assembly is provided comprising a locking device, a locking (member or component) held (seated/positioned) within the locking device for releasably coupling a second device that is receivable by the locking device, to the locking device, wherein the locking component has a first state (or configuration) and a second state (or configuration), wherein the locking component is moveable from a first state into a second state upon insertion of the second device into the locking device to allow passage of the second device there-through and the locking component is moveable thereafter into its first state to couple the second device to the locking device.

In some embodiments, a locking system is provided comprising a locking device, a locking (member or component) held (seated/positioned) within the locking device, and A coupling member held within the first mating member, and a second device that is receivable by locking device to be releasably secured thereto by the locking member, wherein the locking component is moveable from its first state into its second state upon insertion of the second device into the locking device to allow passage of the second device there-through and the locking component is moveable thereafter into its first state to couple the second device to the locking device.

### Alternatives

In some embodiments, a releasable coupling mechanism is provided comprising A housing defining a primary mating member [defining an opening] for receiving a secondary mating member to be coupled thereto, an elastically deformable a retaining component [or member] seated [or positioned] within the housing, for releasably coupling the secondary mating member to the housing upon insertion thereof into the housing, wherein the elastically deformable retaining component has a first engaging configuration and a second non-engaging configuration, wherein the elastically deformable retaining component is moveable into its second non-engaging configuration upon insertion of the secondary mating member into the housing to enable the secondary mating member to advance into the housing and is moveable thereafter into its first engaging configuration to couple the secondary mating member to the primary mating member preventing [the secondary mating member to be detachable therefrom, preventing disengagement thereof in the absence of force to prevent the secondary mating member to be removable from the opening thereof].

In some such embodiments retaining component comprises a deflectable member and is deflectable into its second non-engaging configuration upon insertion of the secondary mating member and is capable of returning to its original first engaging configuration thereafter. In some such embodiments, the retaining component comprises a flexible retaining component. In some examples of the releasable coupling mechanism, the retaining component comprises an elastic retaining component. In some examples, retaining component comprises an elastically deformable retaining component.

In some embodiments, the retaining component is contained within the housing so it is substantially enclosed by the housing. In some examples, the housing comprises a cap and a cover, defining a cavity or space therein, and the retaining component is seated or positioned within the cavity or space defined by the housing. In some examples, the retaining component is coupled to the housing. In some examples, the retaining component is attached to the housing. In some examples, the retaining component is attached to the housing at least at an end or point thereof. In some examples, the retaining component is formed integrally with the housing. In some examples, the retaining component comprises at least one retaining arm or member or in other words one or more retaining arms or members.

In some examples, the at least one retaining arm [or member], wherein the one or more retaining arms or [members] terminate in one or more snaps. In some examples, the at least one retaining arm [or member], wherein the one or more retaining arms or [members] comprises one or more cantilevers. In some examples, the one or more cantilevers comprise a straight cantilever. In In some examples, he one or more cantilevers comprise a u-shaped cantilever.

In some embodiments of the u-shaped cantilever, the u-shaped cantilever in its first engaging configuration comprises a substantially u- shaped configuration and wherein the retaining component in its second non-engaging configuration comprises a substantially compressed u-shaped configuration, Wherein the elastically deformable retaining component is moveable into its second non-engaging configuration comprising the substantially compressed u- shaped configuration upon insertion of the secondary mating member into the housing to enable the secondary mating member to advance into the housing and is moveable thereafter into its first engaging configuration comprising the substantially u-shaped configuration to couple the secondary mating member to the primary mating member.

In some embodiments, wherein the u-shaped cantilever in its first engaging configuration comprises a substantially u- shaped configuration and wherein the retaining component in its second non-engaging configuration comprises a substantially expanded shaped configuration, wherein the elastically deformable retaining component is moveable into its second non-engaging configuration comprising the substantially expanded u- shaped configuration upon insertion of the secondary mating member into the housing to enable the secondary mating member to advance into the housing and is moveable thereafter into its first engaging configuration comprising the substantially u-shaped configuration to couple the secondary mating member to the primary mating member.

In some embodiments, the retaining component is retained [or held loosely] within the housing to be constrained therein or restrained. In other words it is substantaially not coupled thereto or affixed thereto/ not rigidly fastened or securely attached. In some such examples, the retaining component is free floating within the housing, ie. Some embodiments provide comprises a free floating retaining component that is free floating within the housing. In some such examples, the retaining component is moveable in a direction that is in a plane that is perpendicular to the direction of advancement of secondary mating member into the housing for insertion therein.

In some embodiments, the retaining component is moveable in a direction that is in a plane that is perpendicular to the direction of advancement of secondary mating member into the housing for insertion therein. In some such examples, the elastically deformable retaining component comprises a substantially disc shaped structure. In some such examples, the elastically deformable retaining component comprises a substantially oval disc shaped structure. In some embodiments, the retaining component is moveable in a direction that is in a plane that is in plane with the direction of advancement of secondary mating member into the housing for insertion therein. In some embodiments, the retaining component is moveable in a plane that is perpendicular with the direction of advancement of secondary mating member into the housing for insertion therein. In some such embodiments, the retaining component is moveable [radially] in a radial direction.

In some embodiments, the retaining component is biased in its first engaging configuration, wherein upon insertion of the secondary mating member into the housing [the secondary mating member exerts an outward radial force against the retaining component whereby] the retaining component is moveable into its second engaging configuration to enable the secondary mating member to advance into the housing, In some embodiments, the retaining component is independently moveable with respect to the housing. In some embodiments, the housing comprises a cap in other words housing base portion and a cover or hub cap, defining a cavity [or space] therein for holding the retaining component therein free from engagement therein.

In some embodiments a releasable coupling mechanism is provided comprising, A coupling comprising a primary receiving hub [defining an opening] for receiving an advancing secondary hub for insertion therein, and an independently moveable coupling/gripping component seated within a cavity of the primary hub and enclosed therein, the independently moveable gripping component having an engaging configuration and a non-engaging configuration, wherein upon insertion of the secondary hub into the primary hub, the independently moveable gripping component is initially moveable into its non-engaging configuration to enable advancement of [an engaging portion of] the secondary hub, and is moveable thereafter into its engaging configuration for gripping/grabbing the portion of the secondary hub to enable coupling thereby preventing disengagement thereof [preventing the secondary hub from being removed from the opening in the absence of force], and wherein upon retraction of the secondary hub from the primary hub, the independently moveable gripping component is moveable into its non-engaging configuration to enable disengagement thereof.

In one example, the independently moveable gripping component comprises a flexible gripping component. In one example, the independently movable gripping component comprises a resilient gripping component. In one example, the independently moveable gripping component is radially moveable.

In one broad aspect, embodiments of the present invention comprise a releasable locking mechanism for releasably coupling two medical devices, the releasable locking mechanism comprising, a first handle portion defining a housing comprising an opening for receiving a part of a second handle portion therein, the second handle portion comprising a second handle wider portion and a second handle groove portion, a flexible locking member or component that is seated within the first handle housing for releasably coupling the second handle portion to the first handle portion, the flexible locking member or component having a first locking configuration and a second non-locking configuration, Wherein upon initial advancement of the second handle portion into the first handle portion, the flexible locking member or component is moveable outwards from its first locking configuration into its second non-locking configuration out of the path of the second handle wider portion upon interaction therewith, and upon further advancement of the second handle portion into the first handle portion, the flexible locking member or component is moveable inwards into the second handle groove portion from its second non-locking configuration into its first locking configuration, in order to releasably couple the dilator hub to the sheath hub, whereby the flexible locking member or component is moveable into its second non-locking configuration upon exertion of force as the dilator hub is pulled to enable disengagement of the releasable locking mechanism.

In some examples, the the flexible locking member or component comprises a resilient locking member or component. In a further broad aspect, embodiments of the present invention comprise a releasable locking mechanism for releasably coupling two medical devices, the releasable locking mechanism comprising, a first handle portion defining a housing comprising an opening for receiving a part of a second handle portion therein, the second handle portion comprising a second handle wider portion and a second handle groove portion, a moveable locking member or component that is seated within the first handle housing for releasably coupling the second handle portion to the first handle portion, the moveable locking member or component having a first locking configuration and a second non-locking configuration, wherein upon initial advancement of the second handle portion into the first handle portion, the moveable locking member or component is moveable outwards from its first locking configuration into its second non-locking configuration out of the path of the second handle wider portion upon interaction therewith, and upon further advancement of the second handle portion into the first handle portion, the moveable locking member or component is moveable inwards into the second handle groove portion from its second non-locking configuration into its first locking configuration, in order to releasably couple the dilator hub to the sheath hub, whereby the moveable locking member or component is moveable into its second non-locking configuration upon exertion of force as the dilator hub is pulled to enable disengagement of the releasable locking mechanism.

In still further embodiments, a releasable locking mechanism is provided for releasably coupling two devices, the releasable locking mechanism comprising,
A first receiving device defining a housing, the housing defining an opening for receiving an second insertion device there-through, the second insertion device having a first diameter that is a smaller effective diameter than the opening enabling it to be advanced into the receiving first device, a means for increasing the effective diameter of the second device, the means comprising a moveable and/or flexible locking member or component, Wherein upon insertion of the second device into the first device through the opening thereof, the moveable and/or flexible locking component is moveable to around the second insertion device creating an interference fit/or interacts with to be coupled to the incoming insertion second device that is inserted there-through, thereby creating a larger effective diameter of the second device, thereby creating an effective diameter of the second device, that is larger than the opening of the first device, thereby preventing it from exiting therefrom. Preventing or blocking the movement of dilator hub out of the sheath hub in the absence of force, thereby preventing the dilator hub from exiting.

In some embodiments, a releasable locking mechanism is provided for releasably coupling two devices, the releasable locking mechanism comprising, A sheath hub defining an opening for receiving a dilator hub there-through, a means for increasing an effective diameter of the dilator hub comprising a moveable and/or flexible locking member or component, wherein the dilator hub has a smaller effective diameter than the opening going into the to enable advancement into the sheath hub and once positioned inside the sheath hub, and wherein once the dilator hub is advanced into the sheath hub, the moveable and/or flexible locking component moves around the dilator hub creates an interference fit/or interacts with the incoming dilator hub preventing it from exiting from the opening, thereby creating a larger effective diameter on the dilator hub that is larger than the opening within the sheath hub, thereby preventing/blocking the movement of dilator hub out of the sheath hub in the absence of force, preventing the dilator hub from exiting.

As a further overview, some embodiments of the present invention provide a novel coupling for two devices. This may be advantageous in scenarios such as when a dilator snaps into a sheath hub to connect the two devices. This is generally done by the user on the proximal end which is the user interface. By connecting the two devices proximally, it ensures the distal portions remain fixed in the correct position while the user guides the sheath and dilator in the patient anatomy.

Inventors of the present invention have invented coupling mechanisms that attempt to overcome problems associated with prior art mechanism. Problems associated with existing devices are that they have snaps which degrade with use, have insufficient retention force, provide insufficient tactile feedback, or generate debris. These snaps typically embody a rigid ring or bump on the dilator hub that must press into a mating feature on the sheath hub.

Additionally there is a need in the art for the user needs to have the ability to rotate the connected devices using either the sheath hub or dilator handle, and/or additionally to allow a dilator (such as a stiff dilator) to be oriented in the same direction as the curve of the sheath.

Inventors of the present invention have developed a snap member with inherent flexibility, which allows the snap to be/become substantially robust. Additionally a coupling mechanisms is provided that provides a rotational key, for example that has been added to the dilator and sheath hubs. In some such embodiments, this ensures proper alignment and the ability to transmit torque. Thus some embodiments of the present invention, provide a coupling mechanism that provide a) a snap mechanism with inherent flexibility, and/or b) providing mechanical engagement between the dilator and sheath hubs which can transfer torque and which provides for consistent alignment.

In some embodiments, the direction of the sheath curve is indicated by the side port on the sheath hub and is controlled by rotating the sheath hub.

In one broad aspect, embodiments of the present invention comprise a releasable locking mechanism for releasably coupling two medical devices, the releasable locking mechanism comprising: a first handle portion defining a housing comprising an opening for receiving a part of a second handle portion therein, the second handle portion comprising a second handle wider portion and a second handle groove portion; a flexible locking member or component that is seated within the first handle housing for releasably coupling the second handle portion to the first handle portion; the flexible locking member or component having a first locking configuration and a second non-locking configuration; Wherein upon initial advancement of the second handle portion into the first handle portion, the flexible locking member or component is moveable outwards from its first locking configuration into its second non-locking configuration out of the path of the second handle wider portion upon interaction therewith, and upon further advancement of the second handle portion into the first handle portion, the flexible locking member or component is moveable inwards into the second handle groove portion from its second non-locking configuration into its first locking configuration, in order to releasably couple the dilator hub to the sheath hub, whereby the flexible locking member or component is moveable into its second non-locking configuration upon exertion of force as the dilator hub is pulled to enable disengagement of the releasable locking mechanism.

In a further broad aspect, embodiments of the present invention comprise a releasable locking mechanism for releasably coupling two medical devices, the releasable locking mechanism comprising: a first handle portion defining a housing comprising an opening for receiving a part of a second handle portion therein, the second handle portion comprising a second handle wider portion and a second handle groove portion; a moveable locking member or component that is seated within the first handle housing for releasably coupling the second handle portion to the first handle portion; the moveable locking member or component having a first locking configuration and a second non-locking configuration; Wherein upon initial advancement of the second handle portion into the first handle portion, the moveable locking member or component is moveable outwards from its first locking configuration into its second non-locking configuration out of the path of the second handle wider portion upon interaction therewith, and upon further advancement of the second handle portion into the first handle portion, the moveable locking member or component is moveable inwards into the second handle groove portion from its second non-locking configuration into its first locking configuration, in order to releasably couple the dilator hub to the sheath hub, whereby the moveable locking member or component is moveable into its second non-locking configuration upon exertion of force as the dilator hub is pulled to enable disengagement of the releasable locking mechanism.

As a feature of this broad aspect, the means for increasing the effective diameter of the dilator hub comprises a moveable and/or flexible locking member or component, wherein the dilator hub has a smaller effective diameter going into the sheath hub and once positioned inside the sheath hub the moveable and/or flexible locking component moves around the dilator hub creates an interference fit/or interacts with the incoming dilator hub preventing it from exiting, by creating a larger effective diameter on the dilator hub, where the effective diameter of the dilator hub is larger than the opening within the sheath hub preventing/blocking the movement of dilator hub out of the sheath hub in the absence of force, thereby preventing the dilator hub from exiting.

The embodiment(s) of the invention described above are intended to be exemplary only. The scope of the invention is therefore intended to be limited solely by the scope of the appended claims.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination.

Although the invention has been described in conjunction with specific embodiments thereof, it is evident that many alternatives, modifications and variations will be apparent to those skilled in the art. Accordingly, it is intended to embrace all such alternatives, modifications and variations that fall within the broad scope of the appended claims. Citation or identification of any reference in this application shall not be construed as an admission that such reference is available as prior art to the present invention.

## Claims

1. A releasable coupling mechanism (300) for medical devices comprising:
a substantially oval disc-shaped coupling member (1000) configured to be associated with a first mating member (100) for releasably coupling a second mating member (200), receivable by the first mating member (100), to the first mating member (100); wherein the substantially oval disc-shaped coupling member (1000) has a first state (1000A) and a second state (1000B); and
wherein the substantially oval disc-shaped coupling member (1000) is moveable from the first state (1000A) to the second state (1000B) upon insertion of the second mating member (200) into the first mating member (100) to allow passage of the second mating member (200) and is moveable thereafter from the second state (1000B) to the first state (1000A) to releasably couple the second mating member (200) to the first mating member (100), **characterized in that**:
the substantially oval disc-shaped coupling member (1000) has in its first state (1000A) a first engaging configuration having a substantially oval disc-shaped configuration and in its second state (1000B) a non-engaging configuration having a substantially round or circular disc-shape configuration,
the substantially oval disc-shaped coupling member (1000) comprises a pair of cantilevers (1200) that are coupled together at each of their respective ends by an arcuate segment, the pair of cantilevers (1200) including snaps (1330, 1630) comprising a first ramp (1333A, 1633A) and a second ramp (1333B, 1633A), wherein a first ramp angle of the first ramp (1333A, 1633A) defines an insertion force, and a second ramp angle of the second ramp (1333B, 1633A) defines a removal force, each of the first and second ramp angles creating a component of force that compresses the snaps, the substantially oval disc-shaped coupling member (1000) elastically deforming to move between the first state (1000A) and the second state (1000B) in response to overcoming the removal force or the insertion force.

2. The releasable coupling mechanism (300) of claim 1, wherein the substantially oval disc-shaped coupling member (1000) is positionable inside the first mating member (100).

3. The releasable coupling mechanism (300) of claim 1 or claim 2, wherein the second mating member (200) is a dilator, and/or the first mating member (100) is a sheath.

4. The releasable coupling mechanism (300) of claim 1, wherein the pair of cantilevers (1200) comprise two substantially straight segments (S) that are parallel to flat surfaces (408) of the first mating member.

5. The releasable coupling mechanism (300) of any one of claims 1 to 4, wherein the disc-shaped coupling member (1000) is deflectable radially to move between the first state (1000A) and the second state (1000B) on insertion of the second mating member (200) into the first mating member (100).

6. The releasable coupling mechanism (300) of any one of claims 1 to 5, wherein the substantially oval disc-shaped coupling member (1000) is configured to generate an audible feedback upon movement between the first state (1000A) and the second state (1000B).

7. The releasable coupling mechanism (300) of any one of claim 1 to 6, further comprising a rotational locking mechanism (400).

8. The releasable coupling mechanism (300) of claim 7, wherein the rotational locking mechanism (400) comprises a first corresponding engagement feature of the first mating member (100) and a second corresponding engagement feature of the second mating member (200), wherein the first and second corresponding features enable rotational locking.

9. The releasable coupling mechanism (300) of claim 1, wherein the substantially oval disc-shaped coupling member (1000) comprises a flexible snap fit mechanism at an interface between mating portions of the first mating member (100) and the second mating member (200).

10. The releasable coupling mechanism (300) of claim 1 to 9, wherein the first ramp (1333A, 1612) and the second ramp (1333B, 1613) are equivalent.

11. The releasable coupling mechanism (300) of claim 1 to 10, wherein the first ramp (1333A, 1612) and the second ramp (1333B, 1613) have different ramp angles, defining different removal and insertion forces.

12. A releasable coupling system (500) comprising:
a first mating member (100);
a coupling mechanism, according to any one of claims 1 to 11, the coupling mechanism including the substantially oval disc-shaped coupling member (1000) associated with the first mating member (100);
a second mating member (200) that is receivable by the first mating member (100) to be secured thereto by the disc-shaped coupling member.

## Patentansprüche

1. Lösbarer Kupplungsmechanismus (300) für medizinische Vorrichtungen, umfassend:
ein im Wesentlichen ovales, scheibenförmiges Kopplungselement (1000), das eingerichtet ist, mit einem ersten Verbindungselement (100) verbunden zu werden, um ein zweites Verbindungselement (200), das vom ersten Verbindungselement (100) aufgenommen werden kann, lösbar mit dem ersten Verbindungselement (100) zu koppeln; wobei das im Wesentlichen ovale, scheibenförmige Kopplungselement (1000) einen ersten Zustand (1000A) und einen zweiten Zustand (1000B) aufweist; und
wobei das im Wesentlichen ovale, scheibenförmige Kopplungselement (1000) beim Einführen des zweiten Verbindungselements (200) in das erste Verbindungselement (100) vom ersten Zustand (1000A) in den zweiten Zustand (1000B) bewegt werden kann, um den Durchgang des zweiten Verbindungselements (200) zu ermöglichen, und anschließend vom zweiten Zustand (1000B) in den ersten Zustand (1000A) bewegt werden kann, um das zweite Verbindungselement (200) lösbar mit dem ersten Verbindungselement (100) zu koppeln, **dadurch gekennzeichnet, dass**:
das im Wesentlichen ovale, scheibenförmige Kopplungselement (1000) in seinem ersten Zustand (1000A) eine erste Eingriffskonfiguration mit einer in der Wesentlichen ovalen, scheibenförmigen Konfiguration und in seinem zweiten Zustand (1000B) eine Nicht-Eingriffskonfiguration mit einer in der Wesentlichen runden oder kreisförmigen, scheibenförmigen Konfiguration aufweist,
das im Wesentlichen ovale, scheibenförmige Kopplungselement (1000) ein Paar Ausleger (1200) umfasst, die an ihren jeweiligen Enden durch ein bogenförmiges Segment miteinander verbunden sind, wobei das Paar Ausleger (1200) Schnappelemente (1330, 1630) aufweist, die eine erste Rampe (1333A, 1633A) und eine zweite Rampe (1333B, 1633A) aufweisen, wobei ein erster Rampenwinkel der ersten Rampe (1333A, 1633A) eine Einführkraft definiert und ein zweiter Rampenwinkel der zweiten Rampe (1333B, 1633A) eine Entnahme-Kraft definiert, wobei sowohl der erste als auch der zweite Rampenwinkel eine Kraftkomponente erzeugen, die die Schnappelemente zusammendrückt, wobei sich das im Wesentlichen ovale, scheibenförmige Kopplungselement (1000) elastisch verformt, um sich als Reaktion auf das Überwinden der Entnahme-Kraft oder der Einführkraft zwischen dem ersten Zustand (1000A) und dem zweiten Zustand (1000B) zu bewegen.

2. Lösbarer Kupplungsmechanismus (300) nach Anspruch 1, wobei das im Wesentlichen ovale, scheibenförmige Kopplungselement (1000) innerhalb des ersten Verbindungselements (100) positionierbar ist.

3. Lösbarer Kupplungsmechanismus (300) nach Anspruch 1 oder Anspruch 2, wobei das zweite Verbindungselement (200) ein Dilatator ist und/oder das erste Verbindungselement (100) eine Hülle ist.

4. Lösbarer Kupplungsmechanismus (300) nach Anspruch 1, wobei das Paar Ausleger (1200) zwei im Wesentlichen gerade Segmente (S) umfasst, die parallel zu ebenen Oberflächen (408) des ersten Verbindungselements verlaufen.

5. Lösbarer Kupplungsmechanismus (300) nach einem der Ansprüche 1 bis 4, wobei das scheibenförmige Kopplungselement (1000) radial auslenkbar ist, um sich beim Einführen des zweiten Verbindungselements (200) in das erste Verbindungselement (100) zwischen dem ersten Zustand (1000A) und dem zweiten Zustand (1000B) zu bewegen.

6. Lösbarer Kupplungsmechanismus (300) nach einem der Ansprüche 1 bis 5, wobei das im Wesentlichen ovale, scheibenförmige Kopplungselement (1000) eingerichtet ist, bei einer Bewegung zwischen dem ersten Zustand (1000A) und dem zweiten Zustand (1000B) ein akustisches Rückmeldesignal zu erzeugen.

7. Lösbarer Kupplungsmechanismus (300) nach einem der Ansprüche 1 bis 6, der ferner einen Drehverriegelungsmechanismus (400) umfasst.

8. Lösbarer Kupplungsmechanismus (300) nach Anspruch 7, wobei der Drehverriegelungsmechanismus (400) ein erstes entsprechendes Eingriffsmerkmal des ersten Verbindungselements (100) und ein zweites entsprechendes Eingriffsmerkmal des zweiten Verbindungselements (200) umfasst, wobei das erste und das zweite entsprechende Eingriffsmerkmal eine Drehverriegelung ermöglichen.

9. Lösbarer Kupplungsmechanismus (300) nach Anspruch 1, wobei das im Wesentlichen ovale, scheibenförmige Kopplungselement (1000) einen flexiblen Schnappverschlussmechanismus an einer Schnittstelle zwischen verbindenden Teilen des ersten Verbindungselements (100) und des zweiten Verbindungselements (200) aufweist.

10. Lösbarer Kupplungsmechanismus (300) nach einem der Ansprüche 1 bis 9, wobei die erste Rampe (1333A, 1612) und die zweite Rampe (1333B, 1613) equivalent sind.

11. Lösbarer Kupplungsmechanismus (300) nach einem der Ansprüche 1 bis 10, wobei die erste Rampe (1333A, 1612) und die zweite Rampe (1333B, 1613) unterschiedliche Rampenwinkel aufweisen, wodurch unterschiedliche Entnahme- und Einführkräfte definiert werden.

12. Lösbarer Kupplungssystem (500), umfassend:
ein erstes Verbindungselement (100);
einen Kupplungsmechanismus nach einem der Ansprüche 1 bis 11, wobei der Kupplungsmechanismus das im Wesentlichen ovale, scheibenförmige Kopplungselement (1000) umfasst, das dem ersten Verbindungselement (100) zugeordnet ist;
ein zweites Verbindungselement (200), das in das erste Verbindungselement (100) aufgenommen werden kann, um daran mittels des scheibenförmigen Kopplungselements befestigt zu werden.

## Revendications

1. Mécanisme de couplage libérable (300) pour dispositifs médicaux comprenant :
un élément de couplage en forme de disque sensiblement ovale (1000) configuré pour être associé à un premier élément d'accouplement (100) pour coupler de manière libérable un second élément d'accouplement (200), qui peut être reçu par le premier élément d'accouplement (100), au premier élément d'accouplement (100) ; dans lequel l'élément de couplage en forme de disque sensiblement ovale (1000) présente un premier état (1000A) et un second état (1000B) ; et
dans lequel l'élément de couplage en forme de disque sensiblement ovale (1000) peut être déplacé depuis le premier état (1000A) jusqu'au second état (1000B) suite à l'insertion du second élément d'accouplement (200) à l'intérieur du premier élément d'accouplement (100) afin de permettre le passage du second élément d'accouplement (200) et peut ensuite être déplacé depuis le second état (1000B) jusqu'au premier état (1000A) afin de coupler de manière libérable le second élément d'accouplement (200) au premier élément d'accouplement (100),
**caractérisé en ce que** l'élément de couplage en forme de disque sensiblement ovale (1000) présente, dans son premier état (1000A), une première configuration d'engagement qui a une conformation en forme de disque sensiblement ovale et dans son second état (1000B), une configuration de non-engagement qui a une conformation en forme de disque sensiblement ronde ou circulaire ; et
l'élément de couplage en forme de disque sensiblement ovale (1000) comprend une paire de bras en porte-à-faux (1200) qui sont couplés entre eux au niveau de chacune de leurs extrémités respectives par un segment en arc de cercle, la paire de bras en porte-à-faux (1200) incluant des éléments d'encliquetage (1330, 1630) qui comprennent une première rampe (1333A, 1633A) et une seconde rampe (1333B, 1633A) ; dans lequel un premier angle de rampe de la première rampe (1333A, 1633A) définit une force d'insertion et un second angle de rampe de la seconde rampe (1333B, 1633A) définit une force de retrait, chacun des premier et second angles de rampe créant une composante de force qui comprime les éléments d'encliquetage, l'élément de couplage en forme de disque sensiblement ovale (1000) étant déformé élastiquement pour être déplacé entre le premier état (1000A) et le second état (1000B) suite au fait que la force de retrait ou la force d'insertion est vaincue.

2. Mécanisme de couplage libérable (300) selon la revendication 1, dans lequel l'élément de couplage en forme de disque sensiblement ovale (1000) peut être positionné à l'intérieur du premier élément d'accouplement (100).

3. Mécanisme de couplage libérable (300) selon la revendication 1 ou 2, dans lequel le second élément d'accouplement (200) est un dilatateur et/ou le premier élément d'accouplement (100) est une gaine.

4. Mécanisme de couplage libérable (300) selon la revendication 1, dans lequel la paire de bras en porte-à-faux (1200) comprend deux segments sensiblement rectilignes (S) qui sont parallèles à des surfaces planes (408) du premier élément d'accouplement.

5. Mécanisme de couplage libérable (300) selon l'une quelconque des revendications 1 à 4, dans lequel l'élément de couplage en forme de disque (1000) peut être fléchi radialement afin d'être déplacé entre le premier état (1000A) et le second état (1000B) suite à l'insertion du second élément d'accouplement (200) à l'intérieur du premier élément d'accouplement (100).

6. Mécanisme de couplage libérable (300) selon l'une quelconque des revendications 1 à 5, dans lequel l'élément de couplage en forme de disque sensiblement ovale (1000) est configuré pour générer un signal de retour audible suite à un déplacement entre le premier état (1000A) et le second état (1000B).

7. Mécanisme de couplage libérable (300) selon l'une quelconque des revendications 1 à 6, comprenant en outre un mécanisme de verrouillage en rotation (400).

8. Mécanisme de couplage libérable (300) selon la revendication 7, dans lequel le mécanisme de verrouillage en rotation (400) comprend une première caractéristique fonctionnelle d'engagement à coopération mutuelle du premier élément d'accouplement (100) et une seconde caractéristique fonctionnelle d'engagement à coopération mutuelle du second élément d'accouplement (200), et dans lequel les première et seconde caractéristiques fonctionnelles d'engagement à coopération mutuelle permettent un verrouillage en rotation.

9. Mécanisme de couplage libérable (300) selon la revendication 1, dans lequel l'élément de couplage en forme de disque sensiblement ovale (1000) comprend un mécanisme d'encliquetage souple au niveau d'une interface entre des parties d'accouplement du premier élément d'accouplement (100) et du second élément d'accouplement (200).

10. Mécanisme de couplage libérable (300) selon l'une quelconque des revendications 1 à 9, dans lequel la première rampe (1333A, 1612) et la seconde rampe (1333B, 1613) sont équivalentes.

11. Mécanisme de couplage libérable (300) selon l'une quelconque des revendications 1 à 10, dans lequel la première rampe (1333A, 1612) et la seconde rampe (1333B, 1613) présentent des angles de rampe différents, d'où la définition de forces d'insertion et de retrait différentes.

12. Système de couplage libérable (500) comprenant :
un premier élément d'accouplement (100) ;
un mécanisme d'accouplement selon l'une quelconque des revendications 1 à 11, le mécanisme d'accouplement incluant l'élément de couplage en forme de disque sensiblement ovale (1000) qui est associé au premier élément d'accouplement (100) ; et
un second élément d'accouplement (200) qui peut être reçu par le premier élément d'accouplement (100) afin de lui être fixé au moyen de l'élément de couplage en forme de disque sensiblement ovale.
